(19) Europäisches Patentamt
European Patent Office
Office européen des brevets

(11) EP 4 172 190 B1

(12) **EUROPEAN PATENT SPECIFICATION**

(45) Date of publication and mention of the grant of the patent:
**29.01.2025 Bulletin 2025/05**

(21) Application number: **21828231.7**

(22) Date of filing: **18.02.2021**

(51) International Patent Classification (IPC):
***C07K 14/78*** *(2006.01)*

(52) Cooperative Patent Classification (CPC):
(C-Sets available)
**C08J 3/075; C07K 14/78; C08H 1/06; C08J 3/24; C08L 89/06;** C08J 2389/00; C08K 5/3415 (Cont.)

(86) International application number:
**PCT/JP2021/006136**

(87) International publication number:
**WO 2021/261008 (30.12.2021 Gazette 2021/52)**

(54) **MODIFIED GELATINS**

MODIFIZIERTE GELATINEN

GÉLATINES MODIFIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2020 GB 202009772**

(43) Date of publication of application:
**03.05.2023 Bulletin 2023/18**

(73) Proprietor: **FUJIFILM Corporation**
**Tokyo 106-8620 (JP)**

(72) Inventors:
- **VAN BOXTEL, Huibert Albertus**
  **5047 TK Tilburg (NL)**
- **VAN DONGEN, Elisabeth Marianna Wilhelmina Maria**
  **5047 TK Tilburg (NL)**

(74) Representative: **Hoffmann Eitle**
**Patent- und Rechtsanwälte PartmbB**
**Arabellastraße 30**
**81925 München (DE)**

(56) References cited:
**WO-A1-2008/103041 WO-A1-2008/103045**
**US-A1- 2015 071 985**

- **SANDRA VAN VLIERBERGHE ET AL: "Reversible gelatin-based hydrogels: Finetuning of material properties", EUROPEAN POLYMER JOURNAL, PERGAMON PRESS LTD OXFORD, GB, vol. 47, no. 5, 20 February 2011 (2011-02-20), pages 1039 - 1047, XP028191261, ISSN: 0014-3057, [retrieved on 20110224], DOI: 10.1016/ J.EURPOLYMJ.2011.02.015**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**G02B 1/043, C08L 89/06;**
C08K 5/3415, C08L 89/06

## Description

### Technical Field

This invention relates to modified gelatins, to a process for making modified gelatins, to hydrogels, to a process for making hydrogels and to kits suitable for making hydrogels.

### Background Art

Hydrogels are three-dimensional, hydrophilic, polymeric networks capable of absorbing large amounts of water and/or biological fluids. Due to their high water content, porosity and soft consistency, they closely simulate natural, living tissue.

Hydrogels may be used in a number of areas, contact lenses, wound dressings, drug delivery systems, tissue engineering and in hygiene products. Some hydrogels can be used as an effective matrix platform to encapsulate cells in 3D cell cultures.

Synthetic polymers and naturally-derived biopolymers are among the types of hydrogel precursors that are extensively studied. In general, synthetic gel matrices can be easily optimized in mechanical strength and degradation rate. However, synthetic polymer scaffolds like poly(ethylene glycol)-derived hydrogels (PEG-based) lack bio-functionality and do not interact with cells. In contrast, naturally-derived biomaterials such as collagen, gelatins, fibrin, and hyaluronic acid are capable of providing cell interaction sites, but may yield weaker mechanical properties due to the broad mesh size distribution in the hydrogel network. The range of distances between crosslink junctions along the biopolymer backbones is wide for the $\alpha 1$ and $\alpha 2$ chains in gelatins, causing the mechanical properties in the three-dimensional network to become inhomogeneous. Clearly there is a need for a material that combines both biological functionality and synthetic chemical control. Recombinant gelatins offer a preferable combination of control and cell interaction.

A widely-used process to form hydrogels is based on photoinitiated radical polymerization. Mostly using acrylate moieties in poly(ethylene glycol) diacrylate or methacrylated biopolymers like gelatins, hyaluronic acid and dextran. With an added photoinitiator and light exposure the polymerization is initiated and a hydrogel network is formed. When exposed to free radicals, the (meth)acrylates crosslink into chains hundreds to thousands of monomers long by a chain-growth mechanism causing an inhomogeneous network, post-crosslinking hydrogel shrink and optical turbidity. As one of the disadvantages of this polymerization process we have found that the light exposure intensity used is critically determining the resulting hydrogel mechanical properties. Under conditions of a fixed total light exposure, long exposure with lower intensity versus short exposure with high intensity, a variation on final hydrogel compressive modulus of more than a factor 2 difference is found for methacrylated (recombinant) gelatins. The low intensity results in the softest hydrogel and also in a very noticeable hydrogel turbidity. The highest exposure intensity results in the highest rigidity in combination with acceptable optical transparency. Obviously, when cells need to be encapsulated before polymerization high (UV) light exposures are not preferred. Cell viability can be compromised.

Alternatively, it is beneficial to use a mixture of thiols and olefins (with one or more ethylenically unsaturated groups, a.k.a. "ene"s) as precursors in the photoinitiated radical polymerization process. The addition of thiol precursors causes the reaction to have different characteristics from that of (meth)acrylates alone and a number of important advantages. The thiol-ene free radical reactions are not susceptible to oxygen inhibition during hydrogel formation. The photopolymerization kinetics are faster than that of methacrylates and the polymerization needs less photoinitiator to complete. Contrary to the chain-growth mechanism that is seen with acrylates alone the thiol-ene network is formed by a step-growth mechanism, resulting in a homogeneous network and negligible post-gelation shrink. This is beneficial since it should result in less variability in cell response to the mechanical cues of the hydrogel. In addition, in case an application requires hydrogel encapsulated proteins and/or growth factors these molecules are much less degraded by the thiol-ene photopolymerization than by that of the (meth)acrylates as sole precursor types.

However, there can be a number of reasons why photochemical crosslinking may not be the most preferred option. For large volumes the optical path length can become too long causing inhomogeneous exposure intensities. In vivo application target sites may block light from reaching the sample. The cell type(s) that need to be encapsulated may be too sensitive for the required transient photo-radicals, or end-users may not have, or do not find it convenient to use the required exposure equipment. In such cases it is necessary to use a click type reaction that is cytocompatible at the same time.

A preferred click-type reaction to form hydrogels is obtained by using a combination of thiol compounds and compounds with electron-deficient "ene"s like acrylates as hydrogel precursors. Since low molecular weight thiol-containing compounds typically show an unpleasant odor and are usually toxic to cells it is preferred to anchor the thiols covalently to a large molecular weight (bio-)polymer to prevent smell and cytotoxicity. The "ene"-containing compounds on the other hand usually do not smell and low molecular-weight precursors can be used. It is known however that acrylate-containing compounds with molecular weights less than 3,000 Dalton exhibit significant cytotoxicity. This limits the use of

such crosslinkers to applications without encapsulated cells.

In some embodiments it is preferred to avoid the use of synthetic crosslinkers comprising poly(ethylene glycol) and instead modify a gelatin or a polysaccharide with electron-deficient "ene"s like acrylates, methacrylates, acrylamides, methacrylamides, maleimides or vinylsulfones. The addition of methacrylamide or methacrylate groups is done using methacrylic anhydride in aqueous solution in high yield, as is known in the art. Such biopolymer-type crosslinking agents then typically have a high molecular weight. Examples of such crosslinking agents are methacrylated versions of recombinant gelatins (RG), hyaluronan (HA), heparan sulfate (HS), heparin (HP), chondroitin sulfate (CS), dermatan sulfate (DS), and keratan sulfate (KS). The functionality of such biopolymer crosslinking agents is typically higher than 4. In some embodiments the functionality can be at least 10.

In a preferred embodiment of the current invention the hydrogels are formed by a thiol-Michael addition reaction between thiols and electron-deficient "ene"s, without the use of photoinitiators.

WO2008/103041 describes recombinant gelatins for use in applications involving cell attachment, for example in cell culture work and applications involving cell cultures of anchor dependent cells and also in a variety of medical applications. Cell attachment plays an important role in medical applications such as wound treatment (including artificial skin materials), bone and cartilage (re)growth, implantations and artificial blood vessel materials and blocking of attachment receptors of cells.

Van Vlierberghe et al., European Polymer Journal 47 (2011) 1039-1047 discloses a modified gelatin having 350 μmol/g of lysine thiolated with 2-iminothiolane or N-acetyl homocysteine thiolactone.

## Summary of Invention

It has now been found that recombinant gelatins such as those described in WO2008/103041 may be modified to provide materials having useful properties, including use as a water-soluble polymer for the preparation of novel hydrogels.

The present invention provides the following invention.

(1) A modified gelatin comprising:

(a) lysine residues; and
(b) lysine residues comprising a pendent side chain carrying a thiol group;

wherein the modified gelatin comprises at least 50μmoles/g of component (b) and at least 450μmoles/g in total components (a) and (b).

(2) A modified gelatin according to (1) which comprises components (a) and (b) at an average distance of 17 amino acids with a distance standard deviation of 12 amino acids.

(3) A modified gelatin according to (1) or (2) wherein the standard deviation of the distances between components of type (a), between components of type (b) and between components of type (a) and (b) is less than 14 amino acid residues.

(4) A modified gelatin according to any one of (1) to (3) which has a weight-averaged molecular weight from 15,000 to 80,000 Daltons.

(5) A modified gelatin according to any one of (1) to (4) which has an isoelectric point of at least 5.

(6) A modified gelatin according to any one of (1) to (5) in which the percentage of RGD motifs relative the total number of amino acids present in the recombinant gelatin is at least 0.4%.

(7) A modified gelatin according to any one of (1) to (6) which comprises at least two RGD sequences per 250 amino acids.

(8) A modified gelatin according to any one of (1) to (7) which is free from cysteine.

(9) A process for preparing a modified gelatin comprising reacting a recombinant gelatin comprising at least 450μmoles/g of lysine with a thiolating agent, wherein the modified gelatin comprises at least 50μmoles/g of thiol groups derived from the thiolating agent.

(10) A process according to (9) wherein the thiolating agent comprises a 2-iminothiolane and/or DL- N-acetylhomocysteine thiolactone and/or a salt thereof.

(11) A modified gelatin obtainable by a process according to (9) or (10).

(12) A hydrogel obtainable by reacting a modified gelatin according to any one of (1) to (8) or (11) with a crosslinking agent using a click-reaction.

(13) A hydrogel obtainable by reacting a modified gelatin according to (12) where the click-reaction is a thiol-Michael addition reaction.

(14) A hydrogel according to (12) or (13) wherein the crosslinking agent comprises at least two ethylenically unsaturated groups.

(15) A hydrogel according to (12) or (13) or (14) wherein the crosslinking agent comprises at least one maleimide group.

(16) A hydrogel according to any one of (12) to (15) wherein the crosslinking agent comprises at least one maleimide group and at least one acrylate group.

(17) A hydrogel according to any one of (12) to (16) wherein the crosslinking agent comprises one and only one maleimide group and one and only one acrylate group.

(18) A hydrogel according to any one of (12) to (17) wherein the crosslinking agent has an Mw of from 2,000 to 30,000 Daltons.

(19) A hydrogel according to any one of (12) to (18) wherein the number of moles of crosslinking agent which are added to the modified gelatin are insufficient to react with all of the thiol groups present in the modified gelatin.

(20) A hydrogel according to any one of (12) to (19) wherein the number of moles of crosslinking agent which are added to the modified gelatin are sufficient to react with at most 95% of the thiol groups present in the modified gelatin.

(21) A hydrogel according to any one of (12) to (20) which comprises from 0.5 to 15μmoles/g of thiol groups.

(22) A hydrogel according to any one of (12) to (21) which comprises 65 to 97.5 wt% of water.

(23) A hydrogel according to any one of (12) to (21) which has a solids content of up to 10wt%.

(24) A process for preparing hydrogels, comprising reacting a modified gelatin according to any one of (1) to (8) or (11) with a crosslinking agent using a click-reaction.

(25) A kit of parts comprising:

a. a modified gelatin according to any one of (1) to (8) or (11); and
b. a crosslinking agent.

(26) A kit of parts according to (25) wherein the crosslinking agent comprises one and only one maleimide group and one and only one acrylate group.

(27) A kit of parts according to (25) or (26) which further comprises instructions to crosslink the modified gelatin with the crosslinking agent.

**Brief Description of Drawings**

[fig.1]Fig.1 shows calibration curve for the unmodified REC1 gelatin with 644.7 mi-cromoles of lysine per gram using the OPA assay.

[fig.2]Fig.2 shows calibration curve for the unmodified CREC1 gelatin with 400.3 mi-cromoles of lysine per gram using the OPA assay.

**Embodiments for Carrying out the Invention**

According to a first aspect of the present invention there is provided a modified gelatin comprising:

(a) lysine residues; and
(b) lysine residues comprising a pendent side chain carrying a thiol group;

wherein the modified gelatin comprises at least 50μmoles/g of component (b) and at least 450μmoles/g in total components (a) and (b).

The term "comprising" as used in this specification is to be interpreted as requiring the presence of the stated parts, steps or components, but does not exclude the presence of one or more additional parts, steps or components unless explicitly specified.

Reference to an element by the indefinite article "a" or "an" does not exclude the possibility that more than one of the element(s) is present, unless the context clearly requires that there be one and only one of the elements. The indefinite article "a" or "an" thus usually means "at least one".

In this specification all amounts of in μmoles/g of precursor gelatin, modified gelatin and hydrogel are based on the dry weight of the relevant material.

The number of μmoles/g of lysine residues and lysine residues comprising a pendent side chain carrying a thiol group may be determined by the general method described in the Examples section below.

In a second aspect the present invention provides a process for preparing a modified gelatin comprising reacting a recombinant gelatin comprising at least 450μmoles/g of lysine with a thiolating agent, wherein the modified gelatin comprises at least 50μmoles/g of thiol groups derived from the thiolating agent.

The number of μmoles/g of thiol groups derived from the thiolating agent which are present in the modified gelatin may be determined by the method described below in the Examples section.

The recombinant gelatin comprising at least 450μmoles/g of lysine used in the process of the second aspect of the present invention is abbreviated herein to "the precursor gelatin" for brevity. Natural gelatins contain less than 450μmoles/g of lysine.

The precursor gelatin may be obtained by general methods known in the art, for example as described in patent publications EP 0 926 543, EP 1 014 176, WO 01/34646 and WO 2008/03041. The methodology for preparing recombinant gelatins is also described in the publication 'High yield secretion of recombinant gelatins by Pichia pastoris', M.W.T. Werten et al., Yeast 15, 1087-1096 (1999).

The precursor gelatin and the modified gelatin preferably comprise from 150 to 1,000 amino acids. In this specification the terms 'amino acids' and 'amino acid residues' are used interchangeably.

The precursor gelatin and the modified gelatin preferably have a designed molecular weight in the range of 18,000 to 90,000 Daltons. Preferably the production processes to manufacture these precursor gelatins and modified gelatins are well-designed and cause only a minimal number of macromolecular cleavages. As a result the obtained weight-averaged molecular weights ("WAMW") in the range of 15,000 to 80,000 Daltons with a polydispersity index pD of less than 1.2. The WAMW is usually only somewhat lower than the designed MW for recombinant gelatins as a result of minimal polymer chain cleavage.

In contrast to natural gelatins that are extracted from animal tissue, for recombinant gelatins the molecular weight is a result of the designed amino acid sequence. In theory a monodisperse macromolecule is obtained with a defined molecular weight MW. In practice however, due to a slight amount of chain cleavage there is some deviation from the ideal monodispersity. This causes the weight-averaged molecular weight (WAMW) to be lowered somewhat from the designed one and a polydispersity index (pD) that is slightly higher than 1. A designed MW of less than 17,000 or more than 90,000 Daltons is found to give rise to an inefficient production process with low yield and thus is less preferred. In addition, a production batch of modified gelatin characterized by a measured WAMW of more than 80,000 Daltons results in a high solution viscosity during handling of the modified gelatin solution as precursor for the hydrogel formation process. A too high viscosity limits the applicability in applications that require an even leveling of the hydrogel surface.

The precursor gelatin and the modified gelatin preferably have an isoelectric point of at least 5.

Preferably the precursor gelatin is an RGD-enriched recombinant gelatin comprising at least 450μmoles/g of lysine, e.g. a recombinant gelatin comprising at least 450μmoles/g of lysine in which the percentage of RGD motifs relative to the total number of amino acids is at least 0.4 (i.e. an RGD motif which consist of arginine, glycine and aspartate in that order counts as "1" and the number of such RGD motifs relative to the total number of amino acids present in the precursor gelatin is at least 0.4. Thus in the modified gelatin the percentage of RGD motifs relative to the total number of amino acids is also preferably at least 0.4.

When the precursor gelatin or the modified gelatin comprises 350 amino acids or more, preferably each stretch of 350 amino acids comprises at least one RGD motif.

Preferably the precursor gelatin and the modified gelatin comprise at least 450μmoles/g of lysine and at least 0.6% of RGD motifs, more preferably at least 0.8%, especially at least 1.0%, more especially at least 1.2% and most preferably at least 1.5% RGD motifs. A percentage RGD motifs of 0.4 corresponds with at least one RGD sequence per 250 amino acids. The number of RGD motifs is an integer, thus to meet the feature of at least 0.4%, a precursor gelatin or modified gelatin consisting of 251 amino acids must comprise at least two RGD motifs. Preferably the precursor gelatin and the modified gelatin comprise at least two RGD motifs per 250 amino acids, more preferably at least three RGD motifs per 250 amino acids, most preferably at least four RGD motifs per 250 amino acids.

The precursor gelatin and the modified gelatin preferably comprise at least three RGD motifs. In a further embodiment the precursor gelatin and the modified gelatin comprise at least four RGD motifs, preferably at least six, more preferably at least eight, even more preferably at least twelve RGD motifs. Preferably the precursor gelatin and the modified gelatin comprise at most twenty RGD motifs. The precursor gelatin and the modified gelatin preferably comprise lysine, glutamic acid and aspartic acid residues, especially a homogenous distribution of lysine, glutamic acid and aspartic acid residues.

Preferably the precursor gelatin (and the resultant modified gelatin) comprises an even distribution of lysine residues. In this way, reaction with the thiolating agent provides a modified gelatin comprising an even distribution of lysine residues comprising a pendent side chain carrying a thiol group. Then, when the modified gelatin is reacted with a crosslinking agent, a hydrogel results in which crosslinks between modified gelatin molecules are evenly distributed to provide a more homogeneous and mechanically robust hydrogel.

By using recombinant gelatins in the process of the present invention instead of natural gelatins one may ensure that the lysine residues present in the gelatin are more numerous and more evenly distributed/spaced than is found in natural gelatins. Taking into account that there is an upper limit to the preferred amount of lysine residues in the gelatin chain because a too high lysine density implies that other amino acid residues, such as RGD triplets, are inherently lowered in amount, possibly compromising the biological properties of the gelatin. So, there is an optimum in lysine residue content

in the gelatin and the average distance between the lysine along the backbone. As the lysine residues provide anchoring points for the thiolating agents which subsequently crosslink to form hydrogels (see below), the use of recombinant gelatins having the stated content of lysine residues in the process of the second aspect of the present invention provides, after crosslinking, hydrogels having a strong and tunable architecture. Furthermore, a more even distribution of the lysine residues than found in natural gelatins provides resultant hydrogels with a robust architecture and relatively even mesh size. This improvement in architecture is particularly valuable for providing a controlled release of any desirable cells, pharmaceuticals, veterinary products etc. entrapped within the hydrogel.

Bearing in mind the desire for the precursor gelatin to comprise an even distribution of lysine residues for the reasons given above, it is preferred that the precursor gelatin used in the process according to the second aspect of the present invention comprises lysine residues where the average distance between them measured along the backbone is less than 24 with a standard deviation in the distance distribution less than 14 AA. More preferably this average distance between lysine residues is less than 20 AA with a standard deviation less than 14 AA. Most preferred we found an average lysine residue distance of 17 AA with a standard deviation of 12 AA. This, in turn, provides a modified gelatin which comprises components (a) and (b) at an average distance of 17 amino acids with a distance standard deviation of 12 amino acids.

The distance between adjacent lysine residues (ΔLys) is counted by including one of the lysine residues. For example, in the sequence Lys-Arg-Gly-Lys the distance between the two lysine residues is 3 amino acid residues. The average distance between lysine residues in the precursor gelatin ($\Delta Lys_{avg}$) is preferably 10 to 30, more preferably 12 to 25 and especially 14 to 18 amino acid residues (AA). The standard deviation of the distances between lysine residues in the precursor gelatin ($SD_{Lys}$) is preferably 10 to 20 amino acid residues, more preferably 10 to 16 and especially 10 to 13 amino acid residues.

Knowing the amino acid sequence of the precursor gelatin one may calculate $\Delta Lys_{avg}$ and $SD_{Lys}$ as follows: One reads the amino acid sequence from left to right to find the first lysine ($K_1$). Then count the number of residues to get from there to the next lysine ($K_2$) in the sequence ($\Delta Lys_1$), including only the second lysine and not the first. Continue with the sequence from there and count the number of residues to get to the third lysine ($K_3$): $\Delta Lys_2$. Continue with counting until one has the full list of $\Delta Lys_i$. Then calculate the number-average of the listed values $\Delta Lys_{avg}$ and calculate the standard deviation as follows. For each value of $\Delta Lys_i$ calculate the difference between $\Delta Lys_{avg}$ and $ALys_i$ and calculate the square of that value. Do that for the whole list, sum each of the squares to a total, divide that total by the number of listed values (N) and take the square root of that value to obtain the standard deviation in lysine-to-lysine distances $SD_{Lys}$.

[Chem.1]

$$\Delta Lys_{avg} = \sum_i \Delta Lys_i / N$$

$$SD_{Lys} = \sqrt{\left(\sum_i \left(\Delta Lys_i - \Delta Lys_{avg}\right)^2\right)/N}$$

In one embodiment the precursor gelatin may be prepared from another recombinant gelatin comprising at least 30 lysine residues per 1000 amino acid residues.

In a preferred embodiment the precursor gelatin and the modified gelatin are free from cysteine.

In another preferred embodiment the precursor gelatin and the modified gelatin are free from hydroxylysine.

In one preferred embodiment the precursor gelatin comprises or consists of an amino acid sequence having at least 75% amino acid sequence identity to SEQ ID NO: 1, more preferably at least 76%, 78%, 80%, 85%, 90%, 95%, 96%, 98%, 99% sequence identity. In one particularly preferred embodiment the precursor gelatin comprises or consists of the monomer of SEQ ID NO: 1 or a variant thereof, such as an amino acid sequence comprising the above % amino acid sequence identity to SEQ ID NO: 1.

Preparation of the precursor gelatin having the amino acid sequence identity to SEQ ID NO: 1 is described in WO 2008/103041 and the sequence is described therein as SEQ ID NO: 3.

In a second preferred embodiment the precursor gelatin comprises or consisting of an amino acid sequence having at least 75% amino acid sequence identity to SEQ ID NO: 2, more preferably at least 76%, 78%, 80%, 85%, 90%, 95%, 96%, 98%, 99% sequence identity. In one particularly preferred embodiment the precursor gelatin comprises or consists of the monomer of SEQ ID NO: 2 or a variant thereof, such as an amino acid sequence comprising the above % amino acid sequence identity to SEQ ID NO: 2.

Preparation of the precursor gelatin having the amino acid sequence identity to SEQ ID NO: 2 is described in WO 2008/103041 and the sequence is described therein as SEQ ID NO: 5.

In one embodiment the precursor gelatin is free from thiol groups.

As thiolating agent one may use any reagent which incorporates thiol (-SH) groups onto the precursor gelatin or

increases the number of thiol groups present in the precursor gelatin. Typically the thiolating agent comprises a thiol or protected thiol group and a group which is reactive with the amino group present in lysine. The amino group in lysine typically forms a covalent bond with a reactive group present in the thiolating agent and, when the thiol group present in the thiolating agent is protected, the protecting group is removed either during or after reaction of the thiolating agent with the amino group present in the lysine.

Preferred thiolating agents comprise a group of the formula -S-C(=X)- wherein X is S, O or $NR^a$ in which $R^d$ is H or $C_{1-4}$-alkyl. Preferably the S and C atoms shown in the - S-C(=X)- group are part of a ring, e.g. a 4-, 5-, 6- or 7-membered ring.

Examples of suitable thiolating agents include the following (including salt forms of the first and third structure, e.g. the hydrochloric acid salt form):

2-iminothiolane:

[Chem.2]

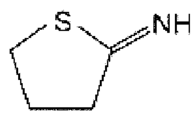

DL-N-acetylhomocysteine thiolactone:

[Chem.3]

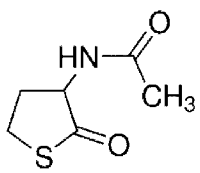

N-Butyryl-DL-homocysteine thiolactone:

[Chem.4]

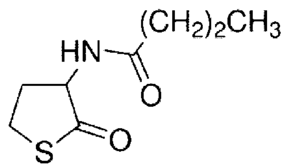

Preferably the thiolating agent comprises a 2-iminothiolane and/or DL-N-acetylhomocysteine thiolactone and/or a salt thereof.

The conditions used for reacting the precursor gelatin with the thiolating agent depend to some extent on the thiolating agent used. However it is preferred that the precursor gelatin is reacted with the thiolating agent in the absence of oxygen gas, for example under a blanket of inert gas (e.g. nitrogen). If desired the reagents used for the reaction may also be degassed to remove oxygen. In this way one avoids or reduces the extent to which the thiol groups in the modified gelatin are oxidized.

Preferably the precursor gelatin is reacted with the thiolating agent at a pH in the range 7 to 12, more preferably 8 to 11 and especially 9 to 10.5. One may achieve the desired pH by performing the reaction in the presence of a base and/or a pH buffer.

If necessary, one may use a buffer to dissolve the thiolating agent. A suitable pH buffer may be prepared by, for example, combining a 0.1 molar sodium carbonate solution (A) with a 0.1 molar sodium bicarbonate solution (B) in a volume ratio of 60 parts A (59.3g) with 40 parts B (39.5g). Such a buffer provides a pH of about 10.0 $\pm$ 0.2 at 25°C.

Another buffer which may be used to dissolve the thiolating agent is phosphate buffer prepared from sodium hydrogen phosphate and water. One may adjust the pH of the phosphate buffer to the desired level (e.g. 8.0) using, for example, hydrochloric acid.

Preferably the precursor gelatin is reacted with the thiolating agent at an elevated temperature, for example a temperature in the range 25 to 60, more preferably 30 to 50 and especially 35 to 45°C.

Preferably the precursor gelatin is reacted with the thiolating agent for a period of 30 minutes to 5 hours, more preferably 1 hour to 4 hours and especially 1 hour to 3 hours.

In a preferred embodiment the precursor gelatin is reacted with the thiolating agent in the presence of a metal chelating agent, for example ethylenediaminetetraacetic acid tetrasodium salt. The metal chelating agent is useful for complexing-out any metals that might otherwise catalyze oxidation of the thiols present in the modified gelatin.

Preferably the modified gelatin according to the first aspect of the present invention is obtainable or obtained by the process according to the second aspect of the present invention.

The preferred molar ratio of thiolating agent to precursor gelatin used in the process of the second aspect of the present invention depends to some extent on the thiolating agent and on the amount of thiol groups desired for the modified gelatin. For example, increasing the ratio of thiolating agent to precursor gelatin may increase the amount of thiol groups provided in the modified gelatin and decreasing the ratio of thiolating agent to precursor gelatin may decrease the amount of thiol groups provided in the modified gelatin.

The extent to which lysine residues which were present in the precursor gelatin have been reacted with the thiolating agent to give the modified gelatin is referred to herein as the DS% of the modified gelatin. For example, if half of the lysine residues in the precursor gelatin reacted with the thiolating agent, the resultant modified gelatin is said to have a DS% of 50%. If all of the lysine residues in the precursor gelatin are reacted with the thiolating agent then the resultant modified gelatin is said to have a DS% of 100%, and so on.

In other words, when the process of the second aspect of the present invention is used to prepare a modified gelatin according to the second aspect of the present invention when the amount of component (a) is identical to the amount of component (b) then the modified gelatin has a DS% of 50%.

The inventors have found that one may make modified gelatins having any desired DS% up to about 100% by following certain formulae. For example, to determine how to make modified gelatins having a desired DS% of Y% by reacting a precursor gelatin with 2-iminothiolane one may perform the following calculation:

The mass of 2-IT in mg required per gram of dry precursor gelatin ($W_{2\text{-}IT}$) to achieve a DS% of Y% is provided by Equation (1):

$$W_{2\text{-}IT} = (Y \times L \times M1)/(74.8 \times 10^6) \text{ Equation (1)}$$

wherein:

Y is the desired % lysine residues to be thiolated;
L is the number of µmoles of lysine present in 1g of the precursor gelatin; and
M1 is the molecular weight of the 2-IT.

When the 2-IT is in the form of its hydrochloride salt, M1 has a value of 137.6.

To determine how to make modified gelatins having a desired DS% of Y% by reacting a precursor gelatin with DL-N-acetylhomocysteine thiolactone (NAHCTL) one may perform the following calculation :
The mass of NAHCTL in mg required per gram of per gram of dry precursor gelatin ($W_{2NAHCTL}$) to achieve a DS% of Y% is provided by Equation (2):

$$W_{NAHCTL} = (Y \times L \times M2)/(53.9 \times 10^6) \text{ Equation (2)}$$

wherein:

Y is the desired % lysine residues to be thiolated;
L is the number of µmoles of lysine present in 1g of the precursor gelatin; and
M2 is the molecular weight of the NAHCTL.

The molecular weight of NAHCTL (M2) is 159.2.

In the above Equations (1) and (2), the weight of gelatin is of course its dry weight (100% solids).

To make hydrogels which are mechanically rigid it is preferred that the modified gelatin has a high DS%, for example a DS% of 30 to 100%, more preferably a DS% of 50 to 100% and especially a DS% of 70 to 100%.

To make hydrogels which are softer it is preferred that the modified gelatins have a lower DS%, for example a DS% of 7 to 50%, more preferably a DS% of 10 to 40% and especially a DS% of 10 to 30%.

Modified gelatins having a DS% in the overlap are between the above two ranges (i.e. DS% of 30 to 70%) have intermediate softness and rigidity.

According to a third aspect of the present invention there is provided a hydrogel obtainable by reacting a modified

gelatin with a crosslinking agent. In this third aspect of the present invention the modified gelatin is as defined in relation to the first aspect of the present invention and/or the modified gelatin is prepared by the process of the second aspect of the present invention.

The crosslinking agent preferably comprises at least two ethylenically unsaturated groups. Thus in a preferred embodiment the thiol groups present in the modified gelatin form covalent bonds with the ethylenically unsaturated groups in the crosslinking agent. In this way, a hydrogel is formed in which multiple molecules of modified gelatin are joined together by the crosslinking agent to form a large structure. When the modified gelatin is reacted with the crosslinking agent under aqueous conditions typically a hydrogel forms.

In a preferred embodiment the crosslinking agent comprises 2 to 4 ethylenically unsaturated groups.

In some embodiments, where gelatins or polysaccharides are modified with (meth)acrylates or (meth)acrylamides the crosslinking agent comprises between 4 and 50 ethylenically unsaturated groups.

In a preferred embodiment the crosslinking agent comprises one or more poly(alkylene oxide) groups, especially one or more poly(ethylene oxide) groups, e.g. of the formula $-(CH_2CH_2O)_m-$ wherein m has a value of 3 to 1,000, especially 3 to 250.

In a preferred embodiment the process according to the third aspect of the present invention is performed in the absence of an initiator (e.g. in the absence of photoinitiators and thermal initiators).

The ethylenically unsaturated groups present in the crosslinking agent may all be chemically identical or the crosslinking agent may comprise two or more different ethylenically unsaturated groups, especially at least two ethylenically unsaturated groups which have different rates of reactivity with thiols.

Preferred ethylenically unsaturated groups are (meth)acrylic, (meth)acrylamide, acrylonitrile, vinylsulfone and maleimide groups.

Examples of suitable (meth)acrylic groups include acrylate ($H_2C=CHCO-$) groups, acrylamide ($H_2C=CHCONH-$) groups, methacrylate ($H_2C=C(CH_3)CO-$) groups and methacrylamide ($H_2C=C(CH_3)CONH-$) groups. Acrylic groups are preferred over methacrylic groups because acrylic groups are more reactive.

Preferred ethylenically unsaturated groups are free from ester groups because this can improve the stability and the pH tolerance of the resultant hydrogel. Ethylenically unsaturated groups which are free from ester groups include (meth)acrylamide groups, vinylsulfones and maleimides ((meth)acrylamide groups and maleimides are especially preferred).

As preferred examples of ethylenically unsaturated groups there may be mentioned groups of the following formulae:

[Chem.5]

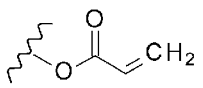
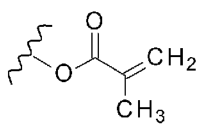
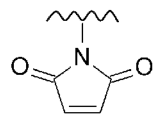
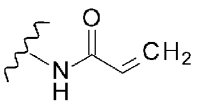
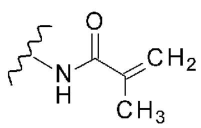

Preferred crosslinking agents comprise a linear, a 3-arm or a 4-arm poly(ethylene glycol) chain and at least two electron-deficient carbon-carbon double bonds.

Preferably the crosslinking agent has an Mw of from 2,000 to 30,000 Daltons, especially from 3,000 to 10,000 Daltons. Examples of preferred crosslinking agents include the following in which 1, m, n, and o each independently have a value of from 3 to 250 and $R_1$, $R_2$, $R_3$ and $R_4$ are each independently H or a carbon-containing moiety:

[Chem.6]

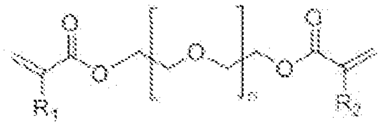

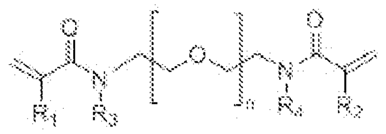

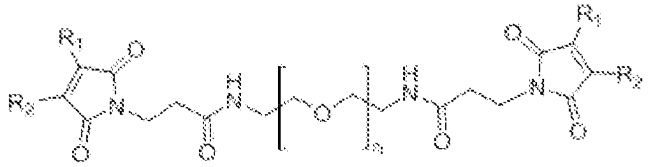

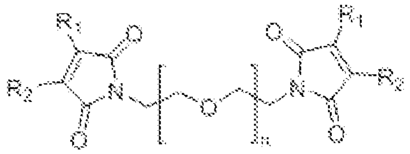

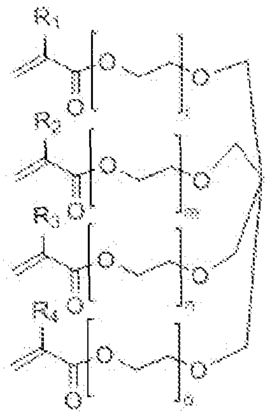

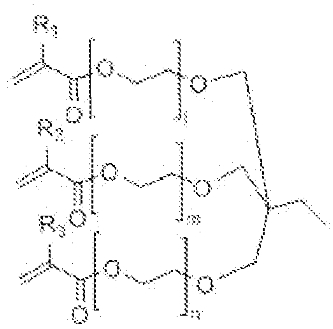

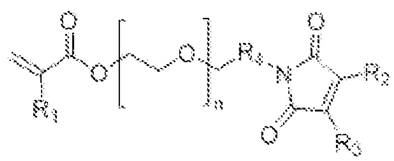

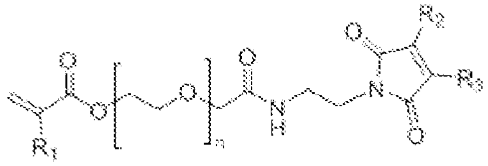

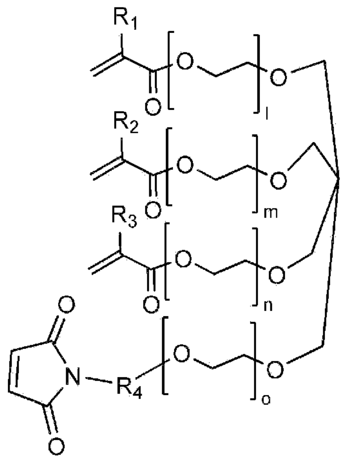

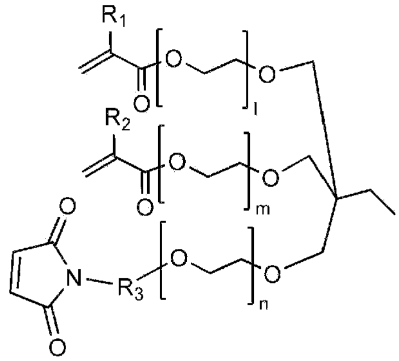

Preferred carbon-containing moieties contain 1 to 4 carbon atoms, e.g. $C_{1-4}$-alky and especially methyl.

In one embodiment the crosslinking agent reacts with the modified gelatin at a rate that allows other components (e.g. cells or pharmaceutically active substances) to become physically entrapped within a network formed by the reaction. Thus a very quick reaction between the modified gelatin according to the first aspect of the present invention and the crosslinking agent (e.g. a reaction time of less than 1 minute) is not preferred. On the other hand a very slow reaction is not preferred either because of the risk of any other components precipitating (e.g. cell sedimentation). Thus, in a preferred embodiment, the time taken for the crosslinking agent to react fully with the modified gelatin is in the range 1 minute to 1 hour, more preferably 5 minutes to 1 hour and most preferably in the range 10 minutes to 45 minutes.

In an embodiment for soft hydrogels, although the crosslinking agent may comprise more than 2 ethylenically unsaturated groups (e.g. 3 or 4) it is preferred that the crosslinking agent comprises 2 and only 2 ethylenically unsaturated groups. Such crosslinking agents can result in hydrogels having particularly useful properties.

In a particularly preferred embodiment the crosslinking agent comprises at least two different ethylenically unsaturated groups, e.g. at least two different ethylenically unsaturated groups which have different rates of reaction with thiol groups, especially one ethylenically unsaturated group which reacts with thiol groups quickly (e.g. a maleimide group) and one ethylenically unsaturated group which reacts with thiol groups relatively slowly (e.g. an acrylate group). In this way, one may achieve an optimal time taken for the crosslinking agent to react fully with the modified gelatin. In this embodiment the crosslinking agent preferably comprises a poly(ethylene glycol) group, e.g. containing 3 to 500 ethylene oxide units.

In one embodiment the crosslinking agent comprises one and only one maleimide group and one and only one acrylate group.

In certain embodiments the crosslinking agent comprises a biopolymer polymer from the groups of polypeptides, gelatins, recombinant gelatins, hyaluronic acid, chondroitin sulphate, keratan sulphate, or dermatan sulphate, modified with ethylenically unsaturated groups. In an embodiment of this invention this crosslinking agent comprises more than 4 ethylenically unsaturated groups. In another embodiment of this invention the crosslinking agent comprises more than 10 ethylenically unsaturated groups.

As an example a gelatin can be modified with methacrylic anhydride to yield a crosslinking agent comprising the modified gelatin with methacrylate and methacrylamide groups.

The recombinant gelatin REC1 can be modified with methacrylic anhydride to yield a crosslinking agent comprising modified REC1 with methacrylamide groups.

Preferably the modified gelatin is reacted with the crosslinking agent at a pH in the range 5.0 to 10.0, more preferably 6.0 to 9.0 and especially 6.8 to 8.0.

Preferably the modified gelatin is reacted with the crosslinking agent in the absence of oxygen gas. For example the modified gelatin is reacted with the crosslinking agent under a blanket of nitrogen gas and optionally any solvents (including water) which come into contact with the modified gelatin have been treated to remove oxygen gas (e.g. by degassing in vacuo and/or by nitrogen purging). In this way one may avoid or lessen the extent of oxidation of the thiol groups present in the modified gelatin.

An advantage of the third aspect of the present invention is that reaction of the modified gelatin with the crosslinking agent does not require the presence of a pH-buffer in a high concentration to counter acid formation during formation of the hydrogel. Thus, in one embodiment, the modified gelatin is reacted with the crosslinking agent in the absence of a pH buffer or in the presence of a pH buffer having a concentration in water of less than 0.1M, more preferably less than 0.05M. Preferably the pH buffer, when present during the reaction of the modified gelatin with the crosslinking agent, maintains a pH in the range 7.0 to 7.8.

Preferably the modified gelatin is reacted with the crosslinking agent at an elevated temperature, for example a

temperature in the range 35 to 39°C, more preferably 36 to 38°C and especially 36.5 to 37.5°C.

In a preferred embodiment the modified gelatin is reacted with the crosslinking agent in the presence of a metal chelating agent, for example ethylenediamine tetraacetic acid and salts thereof. The metal chelating agent is useful for complexing-out any metals that might otherwise catalyze oxidation of the thiol groups present in the modified gelatin.

The crosslinking agent is optionally added to the modified gelatin in the proper stoichiometric ratio, such that all of the thiol groups in the modified gelatin react with the ethylenically unsaturated groups of the crosslinking agent. Preferably, the thiol groups in the modified gelatin are present in (slight) excess to the functional groups of the crosslinking agent. Thus the number of moles of crosslinking agent which is reacted with the modified gelatin is preferably insufficient to react with all of the thiol groups present in the modified gelatin. The latter is preferred because, in this way, the resultant hydrogel formed from the modified gelatin comprises (unreacted or 'free') thiol groups. Such thiol groups are useful as they have anti-oxidant properties, thereby stabilizing any 'payload' which might be entrapped within the hydrogel formed as a result of the crosslinking reaction (e.g. cells and pharmaceutically active substances).

In one embodiment, when the hydrogel is required to comprise thiol groups, the number of moles of crosslinking agent which is reacted with the modified gelatin is preferably sufficient to react with at most 95%, more preferably 20 to 90% and especially 40 to 90% of the thiol groups present in the modified gelatin.

When calculating the amount of crosslinking agent to react with the modified gelatin to achieve the desired degree of crosslinking in the resultant hydrogel one will take account of the number of ethylenically unsaturated groups present in the crosslinking agent. For example, in order to achieve a desired level of crosslinking in the resultant hydrogel (e.g. crosslink 50% of the thiol groups present in the modified gelatin), one would need less crosslinking agent comprising 4 ethylenically unsaturated groups than if a crosslinking agent comprising 3 ethylenically unsaturated groups were used. Similarly, to achieve that same level of crosslinking one would need more crosslinking agent if the crosslinking agent comprised only 2 ethylenically unsaturated groups.

In view of the foregoing, the modified gelatins preferably comprise 300 to 700μmoles/g of thiol groups, more preferably 400 to 700 and especially 450 to 700μmoles/g of thiol groups.

In fact one may tailor the ratio of crosslinking agent to gelatin to achieve harder (more crosslinked) or softer (less crosslinked) hydrogels as desired.

Typically the hydrogel according to the third aspect of the present invention is obtainable by reacting a modified gelatin according to the first aspect of the present invention with a crosslinking agent under aqueous conditions.

Typically the hydrogel takes the form of an open, crosslinked network in which water and, if desired, other substances (e.g. cells or pharmaceuticals) are located within the network. Such hydrogels are useful for their slow release properties, particularly in vivo.

Preferably the hydrogel according to the third aspect of the present invention is obtained by a process in which the weight ratio of modified gelatin to water is in the range in the range 1 to 30%, more preferably 2 to 15, especially 3 to 12% and more especially 5 to 12%. Preferably the water has a very low content of dissolved oxygen, e.g. below 1 part of oxygen per million parts of water, by weight.

In one embodiment the hydrogel has a solids content of up to 8wt%, more preferably 3 to 8wt%. This provides 'soft' hydrogels. To obtain 'hard' hydrogels the hydrogel preferably has a solids content above 8wt%, more preferably from 10 to 30wt%, of the compound according to the second aspect of the present invention. The solids content of the hydrogel may be determined by performing the following calculation:

$$\text{Hydrogel solids content} = [1-[(W1-W2)/W1]] \times 100\%$$

The hydrogels of the present invention can be used to provide spatial and temporal control over the release of various therapeutic agents, including small-molecule pharmaceuticals, macromolecular pharmaceuticals, veterinary treatments and cells. The choice of precursor gelatin, thiolating agent, crosslinking agent and preparation conditions enable the tuning of their physical properties, release rate and capability to protect cells and labile drugs from degradation. The properties of the hydrogels may be tuned to their desired end use.

The hydrogel according to the second aspect of the present invention typically comprises a network of hydrophilic polymer chains derived from the thiol groups in the modified gelatin reacting with the crosslinking agent. The hydrogel is preferably jelly-like. The hydrogel preferably possesses a degree of flexibility which is very similar to that of natural tissue, due in part to its significant water content.

The hydrogel preferably comprises water in an amount of 65 to 97.5 wt%, more preferably 75 to 97 wt% and most preferably 80 to 97 wt%. The water content of the hydrogel may be determined simply by weighing the hydrogel before freeze drying (W1) and after freeze drying (W2) and performing the following calculation:

$$\text{Hydrogel water content} = [(W1-W2)/W1] \times 100\%$$

As mentioned above, if one prepares the hydrogel using insufficient crosslinking agent to react with all of the thiol groups present in the modified gelatin, a hydrogel results which comprises thiol groups. Such thiol groups are useful because they may act as an antioxidant, e.g. to protect any substances (e.g. cells or pharmaceuticals) which may be present in the hydrogel. Thus in a preferred embodiment the hydrogel comprises thiol groups, e.g. derived from a thiolating agent used to prepare the modified gelatin. In a preferred embodiment the hydrogel comprises 0.3 to 60μmoles of thiol groups, more preferably 0.5 to 15μmoles of thiol groups per gram of hydrogel including water present in the hydrogel. One may determine the thiol content of the hydrogel by cutting it into small chunks and performing a thiol quantification assay, e.g. an Ellman's assay.

Depending on the intended use of the hydrogel, one may require a mechanically stiff hydrogel or a soft hydrogel. For example, for medical purposes requiring the encapsulation of cells, it is preferred for the hydrogel to be soft, e.g. having a compressive modulus (E) of less than 100kPa.

In contrast, for hydrogels intended for uses such as corneal tissue replacement the hydrogel preferably has a compressive modulus (E) of 110 to 600kPa, more preferably 200 to 600kPa.

For soft tissue applications and cell therapeutic applications the hydrogel preferably has a compressive modulus (E) of 100 to 20,000Pa, more preferably 200 to 5,000Pa.

The compressive modulus may be measured using a rheometer, e.g. by the method described in the Examples section below.

The preferred compressive modulus may be achieved by selecting precursor gelatins having a desired amount of lysine residues, controlling the extent to which the lysine residues in the gelatin precursor are thiolated and then selecting the extent to which the thiol groups in the modified gelatin are crosslinked with the crosslinking agent. Generally speaking, mechanically stiff hydrogels have a high level of crosslinking and so to make these one will choose a gelatin precursor having a high lysine content, react that with a large amount of thiolating agent to create a modified gelatin having a high DS%, then highly crosslink the modified gelatin having a high DS% using a large amount of crosslinking agent. In contrast, generally speaking, soft hydrogels have a lower level of crosslinking and so to make these one will choose a gelatin precursor having a lower lysine content, react that with a smaller amount of thiolating agent to create a modified gelatin having a low or medium DS%, then crosslink the modified gelatin having the low or medium DS% using a crosslinking agent. Alternatively to make soft hydrogels one may choose a gelatin precursor having a high lysine content, but react only a small proportion of the lysine residues with a thiolating agent to create a modified gelatin having a low thiol content, then crosslink the modified gelatin having the low thiol content using a crosslinking agent.

For making mechanically stiff hydrogels it is preferred that the crosslinking agent has a low molecular weight Mw, for example a Mw in the range 250 to 1,000Da (e.g. MAL-PEG-MAL described in the Examples).

For making hydrogels useful for cell encapsulation the crosslinking agent preferably comprises at least two different ethylenically unsaturated groups which are capable of reacting with thiol groups (e.g. AC-PEG-MAL described in the Examples) and has a molecular weight of at least 3,000 Dalton to minimize cytotoxic effects.

The hydrogels of the present invention may be used in the medical field, e.g. in regenerative cell applications. The hydrogel itself may provide a safe environment for cells and pharmaceutical substances.

The hydrogels of the present invention possess particularly good optical transparency and may be of particular value for preparing contact lenses and repair of tissue in the human eye. Furthermore, they may be used for cell and pharmaceutical delivery in many branches of medicine, including cardiology, oncology, immunology, wound repair and healing and pain management.

The hydrogels of the present invention have excellent optical transparency, high compressive modulus, high rigidity and good storage stability. Furthermore, by crosslinking not all of the thiol groups present in the modified gelatins provides hydrogels which possess anti-oxidant properties, making them of particular value in certain medical applications, e.g. the delivery of therapeutic cells by injection

According to a fourth aspect of the present invention there is provided a contact lens or cornea comprising a hydrogel according to the third aspect of the present invention.

According to a fifth aspect of the present invention there is provided a kit of parts comprising:

(i) a modified gelatin according to the first aspect of the present invention; and
(ii) a crosslinking agent.

In this kit the preferred modified gelatin and crosslinking agent are as described above in relation to the first, second and third aspects of the present invention respectively.

Preferably the kit further comprises instructions to crosslink the modified gelatin with the crosslinking agent, especially in such a way that the number of moles of crosslinking agent which is reacted with the modified gelatin is insufficient to react with all of the thiol groups present in the modified gelatin.

Component (i) is preferably stored in an air-tight container, especially under vacuum or under a blanket of inert gas (e.g. nitrogen gas). In this way one may ensure that the thiol groups present in the modified gelatin do not oxidize during

storage or transportation.

An advantage of the present kits is that the reaction of (i) and (ii) does not cause a substantial pH change and buffers are generally not needed for the preparation of hydrogels. This facilitates the local preparation of hydrogels containing pH-sensitive substances and cells. Cells often cannot tolerate the high ionic strength that is often found with high pH-buffer concentration. The kits may be used by research scientists etc. to prepare hydrogels locally which contain cells or substance of interest to them.

The hydrogels of the present invention are useful for a number of purposes, including the manufacture of contact lenses, corneas, wound dressings, drug delivery systems, cell delivery (including by injection), tissue engineering products and hygiene products. The hydrogels possess particularly good mechanical stability.

A further advantage of the present invention is that the degree of thiolation can be tuned to provide hydrogels having properties tailored for specific end uses. Hydrogels can be prepared comprising specific amounts of free thiol groups (e.g. to provide a hydrogel with anti-oxidant properties) and a desired degree of crosslinking (e.g. to provide a hydrogel with a desired degree of hardness, softness, degradation and release rate).

The invention will now be illustrated with the following non-limiting Examples in which all parts and percentages are by weight unless specified otherwise.

## Examples

The following abbreviations are used in the Examples:

REC1 is a recombinant gelatin having an MW of 51,200 Daltons which comprises 645μmoles/g of lysine and has the amino acid sequence SEQ ID NO: 1 shown below. REC1 was obtained by the method described in WO 2008/103041 and is described therein as SEQ ID NO: 3. The average distance between lysine residues is 16.6 AA with a standard deviation of 11.7 AA.

REC2 is a recombinant gelatin having an MW of 81,100 Daltons which comprises 646μmoles/g of lysine and has the amino acid sequence SEQ ID NO: 2 shown below. REC2 was obtained by the method described in WO 2008/103041 and is described therein as SEQ ID NO: 5. The average distance between lysine residues is 16.8 AA with a standard deviation of 11.8 AA.

CREC1 is a Comparative recombinant gelatin having an MW of 45,000 Daltons which comprises 400μmoles/g of lysine and has the amino acid sequence SEQ ID NO: 3 shown below. CREC1 was obtained by the method described in EP 2,902,413 and is described therein as SEQ ID NO: 1. The average distance between lysine residues is 26.3 AA with a standard deviation of 17.4 AA.

CREC2 is a Comparative recombinant gelatin having an MW of 72,500 Daltons which comprises 442μmoles/g of lysine and has the amino acid sequence SEQ ID NO: 4 shown below. CREC2 was obtained by the method described in US 8,101,205 and is described therein as SEQ ID NO: 1. The average distance between lysine residues is 24.7 AA with a standard deviation of 14.5 AA.

EDTA is ethylenediamine tetraacetic acid, tetrasodium salt dihydrate (a metal sequestering agent to reduce metal-catalysed oxidation of thiol groups) obtained from Sigma-Aldrich.

2-IT is 2-Iminothiolane hydrochloride (a thiolating agent) obtained from Sigma-Aldrich (mwt = 137.63 g/mol).

NAHCTL is DL-N-acetylhomocysteine thiolactone (a thiolating agent) obtained from Sigma-Aldrich (mwt = 159.21 g/mol).

MAnh is methacrylic anhydride (a methacrylating agent) obtained from Sigma-Aldrich (mwt = 154.16 g/mol).

CB10 is a carbonate buffer (pH 10) prepared as follows: A 0.1M sodium carbonate solution was prepared by weighing 2.13g $Na_2CO_3$ into a flask and filling up to a volume of 200ml (a weight of 197.4g) with pure water to give "Solution A". A 0.1M sodium bicarbonate solution was prepared by weighing 1.71g anhydrous $NaHCO_3$ into a flask and filling up to a volume of 200ml (a weight of 200.5g) using pure water to give "Solution B". Solution A (180cm$^3$) and solution B (120cm$^3$) were mixed and filtered to provide the carbonate buffer CB10. CB10 was stored at room temperature in a Corning 500ml bottle until use.

PB8 is a phosphate buffer (pH 8) prepared by mixing dibasic sodium hydrogen phosphate dihydrate (5.37g) in sterile, purified water (150cm$^3$) and adjusting the pH to 8.0 using concentrated hydrochloric acid.

PBS is a buffer prepared by diluting a commercially available "10x concentrated" sterile phosphate buffered saline solution (from Sigma-Aldrich) ten times using sterile purified water.

PEG-AC4 is a crosslinking agent comprising four acrylate groups and four poly(ethylene glycol) chains obtained from Creative PEGWorks, USA, under the product name PSB-423. According to the supplier this crosslinking agent has a Mw 5,000 and has the following general structure:

[Chem.7]

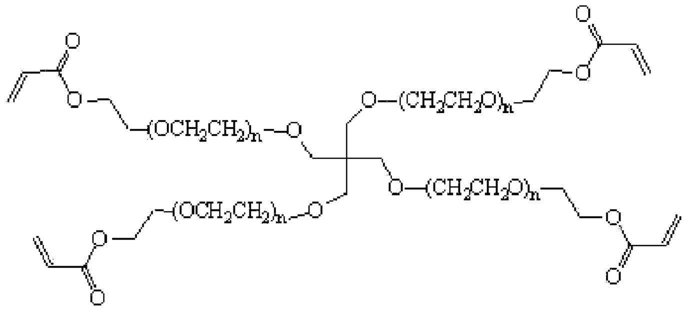

AC-PEG-AC 1 is a crosslinking agent comprising two acrylate groups and one poly(ethylene glycol) chain, obtained from Laysan Bio Inc. under the product name ACRL-PEG-ACRL-3400. According to the supplier this crosslinking agent has a Mw of 3,400 and has the following general structure:

[Chem.8]

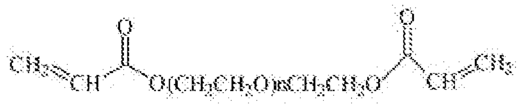

AC-PEG-MAL1 is a crosslinking agent comprising one poly(ethylene glycol) chain one acrylate group and one maleimide group, obtained from Laysan Bio Inc. under the product name ACRL-PEG-MAL-3400. According to the supplier this crosslinking agent has a Mw of 3,400 and has the following general structure:

[Chem.9]

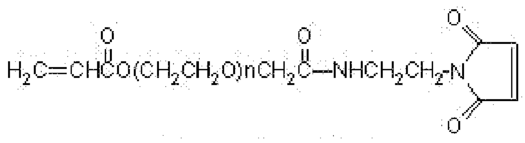

AC-PEG-MAL2 is a crosslinking agent comprising one poly(ethylene glycol) chain one acrylate group and one maleimide group, obtained from Laysan Bio Inc. under the product name ACRL-PEG-MAL-5000. According to the supplier this crosslinking agent has a Mw of 5,000 and has the following general structure:

[Chem.10]

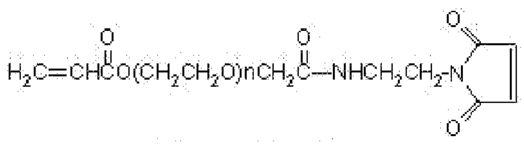

MAL-PEG-MAL is 1,11-bismaleimido-triethyleneglycol, a commercially available crosslinking agent of Mwt 352g/mol from Sigma-Aldrich having the following structure:

[Chem.11]

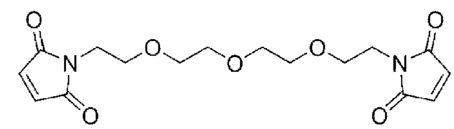

FITC-dextran 150kD, 4kD is Fluorescein isothiocyanate-dextran obtained from Sigma-Aldrich.

REC1 (SEQ ID NO: 1):

GAPGAPGLQGAPGLQGMPGERGAAGLPGPKGERGDAGPKGADGAPGAPGLQGMPGERGAAGLPGPKGERGDAGPKGADGAPGKDGVRGLAGPIGPPGERGAAGLPGPKGERGDAGPKGADGAPGKDGVRGLAGPIGPPGPAGAPGAPGLQGMPGERGAAGLPGPKGERGDAGPKGADGAPGKDGVRGLAGPPGAPGLQGAPGLQGMPGERGAAGLPGPKGERGDAGPKGADGAPGAPGLQGMPGERGAAGLPGPKGERGDAGPKGADGAPGKDGVRGLAGPIGPPGERGAAGLPGPKGERGDAGPKGADGAPGKDGVRGLAGPIGPPGPAGAPGAPGLQGMPGERGAAGLPGPKGERGDAGPKGADGAPGKDGVRGLAGPPGAPGLQGAPGLQGMPGERGAAGLPGPKGERGDAGPKGADGAPGAPGLQGMPGERGAAGLPGPKGERGDAGPKGADGAPGKDGVRGLAGPIGPPGERGAAGLPGPKGERGDAGPKGADGAPGKDGVRGLAGPIGPPGPAGAPGAPGLQGMPGERGAAGLPGPKGERGDAGPKGADGAPGKDGVRGLAGPPG

REC2 (SEQ ID NO: 2):

GAPGAPGLQGAPGLQGMPGERGAAGLPGPKGERGDAGPKGADGAPGAPGLQGMPGERGAAGLPGPKGERGDAGPKGADGAPGKDGVRGLAGPIGPPGERGAAGLPGPKGERGDAGPKGADGAPGKDGVRGLAGPIGPPGPAGAPGAPGLQGMPGERGAAGLPGPKGERGDAGPKGADGAPGKDGVRGLAGPPGAPGLQGAPGLQGMPGERGAAGLPGPKGERGDAGPKGADGAPGAPGLQGMPGERGAAGLPGPKGERGDAGPKGADGAPGKDGVRGLAGPIGPPGERGAAGLPGPKGERGDAGPK

GADGAPGKDGVRGLAGPIGPPGPAGAPGAPGLQGMPGERGAAGLPGPKGERGDAGPKGADGAPGKDGVRGLAGPPGAPGLQGAPGLQGMPGERGAAGLPGPKGERGDAGPKGADGAPGAPGLQGMPGERGAAGLPGPKGERGDAGPKGADGAPGKDGVRGLAGPIGPPGERGAAGLPGPKGERGDAGPKGADGAPGKDGVRGLAGPIGPPGPAGAPGAPGLQGMPGERGAAGLPGPKGERGDAGPKGADGAPGKDGVRGLAGPPGAPGLQGAPGLQGMPGERGAAGLPGPKGERGDAGPKGADGAPGAPGLQGMPGERGAAGLPGPKGERGDAGPKGADGAPGKDGVRGLAGPIGPPGERGAAGLPGPKGERGDAGPKGADGAPGKDGVRGLAGPIGPPGPAGAPGAPGLQGMPGERGAAGLPGPKGERGDAGPKGADGAPGKDGVRGLAGPPGAPGLQGAPGLQGMPGERGAAGLPGPKGERGDAGPKGADGAPGAPGLQGMPGERGAAGLPGPKGERGDAGPKGADGAPGKDGVRGLAGPIGPPGERGAAGLPGPKGERGDAGPKGADGAPGKDGVRGLAGPIGPPGPAGAPGAPGLQGMPGERGAAGLPGPKGERGDAGPKGADGAPGKDGVRGLAGPPG

CREC1 (SEQ ID NO: 3):

GPQGARGQPGVMGFPGPKGANGEPGKAGEKGLPGAPGLRGLPGKDGEAGA
AGPPGPAGPAGERGEQGAPGPPGFQGLPGPPGPPGEGGKPGDQGVPGEAGAPG
LVGPRGERGFPGERGAPGAQGLQGPRGLPGAPGPDGPKGAAGPAGPPGAQGP
PGLQGMPGERGAAGIAGPKGDRGDVGEKGPEGAPGKDGGRGLGGPIGPPGPA
GANGEKGEVGPPGPAGAAGARGAPGERGEAGPPGPAGFAGPPGADGQPGAK
GEQGEAGQKGDAGAPGPQGPGGAPGPQGPAGVAGPKGARGAQGPPGAAGFP
GAAGRVGPPGLQGNPGPPGPPGPAGKDGPKGARGDAGPPGRAGEPGLQGPAG
PPGEKGEPGDDGPPGAEGPPGPQGLAGQRGIVGLPGQRGERGFPGLPGPAGEP
GKQGAPGAAGDRGPPGPVGPPGLAGPAGEPGREGGPGADGPPGRDGAAGVK
GDRGEAGAVGAPGAPGPPGAPGPAGPPGPQGDRGEAGAQGP

CREC2 (SEQ ID NO: 4):

GPPGEPGPTGLPGPPGERGGPGSRGFPGADGVAGPKGPAGERGSPGPAGPKG
SPGEAGRPGEAGLPGAKGLTGSPGSPGPDGKTGPPGPAGQDGRPGPPGPPGAR
GQAGVMGFPGPKGAAGEPGKAGERGVPGPPGAVGPAGKDGEAGAQGPPGPA
GPAGERGEQGPAGSPGFQGLPGPAGPPGEAGKPGEQGVPGDLGAPGPSGPAGE
PGPTGLPGPPGERGGPGSRGFPGADGVAGPKGPAGERGSPGPAGPKGSPGEAG
RPGEAGLPGAKGLTGSPGSPGPDGKTGPPGPAGQDGRPGPPGPPGARGQAGV
MGFPGPKGAAGEPGKAGERGVPGPPGAVGPAGKDGEAGAQGPPGPAGPAGE
RGEQGPAGSPGFQGLPGPAGPPGEAGKPGEQGVPGDLGAPGPSGPAGEPGPTG
LPGPPGERGGPGSRGFPGADGVAGPKGPAGERGSPGPAGPKGSPGEAGRPGEA
GLPGAKGLTGSPGSPGPDGKTGPPGPAGQDGRPGPPGPPGARGQAGVMGFPG
PKGAAGEPGKAGERGVPGPPGAVGPAGKDGEAGAQGPPGPAGPAGERGEQG
PAGSPGFQGLPGPAGPPGEAGKPGEQGVPGDLGAPGPSGPAGEPGPTGLPGPP
GERGGPGSRGFPGADGVAGPKGPAGERGSPGPAGPKGSPGEAGRPGEAGLPG

AKGLTGSPGSPGPDGKTGPPGPAGQDGRPGPPGPPGARGQAGVMGFPGPKGA
AGEPGKAGERGVPGPPGAVGPAGKDGEAGAQGPPGPAGPAGERGEQGPAGSP
GFQGLPGPAGPPGEAGKPGEQGVPGDLGAPGPSGPAGG

MEASUREMENTS

Calculating the Number of μmoles of Lysine per gram of Precursor Gelatin

The number of μmoles/g of lysine present in a recombinant gelatin may be calculated by dividing the number of μmoles of lysine per gram of dry gelatin. More specifically, with knowledge of the amino acid sequence of the recombinant gelatin containing N amino acid residues one may perform the following calculation:

1. First the molecular weights of each amino acid residue ($AA_{i,w}$) are calculated using the residue composition and the known atomic weights of each amino acid, e.g. as tabulated in CRC Handbook of Chemistry and Physics; 78 ed.; Lide, D.R.; Frederikse, H.P.R., Eds.; CRC Press LLC: Boca Raton, 1997.
2. Then, for each recombinant gelatin, the number of residues ($n_i$) for each residue ($AA_i$) is multiplied by its molecular weight to obtain the total molecular weight for each residue type in the gelatin ($n_i\ AA_{i,w}$).

3. All residue type total molecular weights in the gelatin sequence are summed to a value W ($W = \Sigma_i n_i \, AA_{i,w}$).
4. Then (N-1) times the molecular weight of water is subtracted from the value of W to obtain the total gelatin molecular weight $W_{rec}$ ($W_{rec} = W - (N - 1) W_{H20}$).
5. Finally, to obtain the amount of moles lysine per gram gelatin the number of lysine in the gelatin $n_{lys}$ per mole is divided by the gelatin molecular weight $W_{rec}$.

The number of μmoles/g of lysine present in the modified gelatin may be determined in a similar manner of may be calculated from knowing the number of μmoles/g of lysine present in the precursor gelatin and the thiolating agent and DS% of the modified gelatin.

Measurement of the Number of μmoles of Lysine per gram of Precursor Gelatin The number of lysine per gram in precursor gelatins can be determined using spectrophotometric assays with either Ortho-Phthalaldehyde (OPA assay) or trini-trobenzene sulphonic acid (TNBS assay). The OPA assay results are given in the current document. The TNBS assay results match those of the OPA assay and are not given.

Amount of Thiol Groups Derived from the Thiolating Agent
(equivalent to the amount of lysine residues comprising a pendent side chain carrying a thiol group)

Ortho-Phthalaldehyde ("OPA") Assay

The amount of thiol groups (μmoles/g) present in the modified gelatins was determined indirectly using an amine-quantification assay comprising ortho-phthalaldehyde and a low-molecular weight thiol helper-compound similar to 2-mercaptoethanol and fluorescence detection. The reduction in the quantity of lysine amine groups in the modified gelatin, compared to the quantity of lysine amine groups in the original precursor gelatin, correlates to the number of thiol groups incorporate into the modified gelatin as a result of the thiolation reaction.

The measurement of the number of amine groups in the precursor gelatin and the modified gelatin was determined using a Sensolyte™ OPA Protein Quantitation Kit (AnaSpec Catalog # AS-71015) in combination with a fluorescence plate reader (FLX800, Biotek).

In these measurements, (OPA) in the presence of a reducing reagent reacted with the primary amine ($-NH_2$) groups in lysine residues of the precursor gelatins and modified gelatins to form an intense blue fluorescent product. The fluorescence was monitored by exciting the sample under test with an excitation wavelength of 340nm (bandwidth 30nm) and recording the resultant emission at a wavelength of 460nm (bandwidth 40nm). A proprietary, odorless reducing reagent was used.

The assay was performed in accordance with the protocol provided by the manufacturer (Revision 1.1, Oct-2014).

[Chem.12]

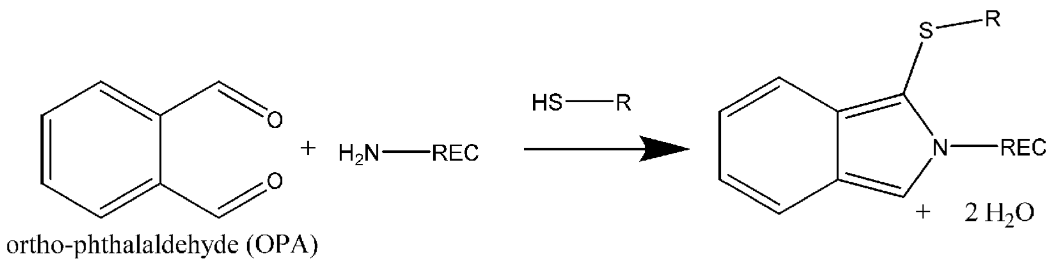

ortho-phthalaldehyde (OPA)

Scheme 1. Chemical reaction of OPA with a primary amine group in a recombinant gelatin ("REC")

OPA assay protocol

First a calibration curve of the precursor gelatin was made according to the published protocol. The fluorescence intensity is measured at Ex/Em=340/460nm using the Biotek plate reader FLX800 using black wells plates. The fluorescence reading from the blank control is the background fluorescence. Subtract this background reading from the readings of the other wells to get relative fluorescence unit (RFU) for all the samples.

For each gelatin a calibration curve is recorded using the assay, see the example for REC1 in the next graph (shown in Figures 1 and 2). From the calibration curves we can deduce that the relative fluorescence intensity per mole lysine is approximated by a value of $(1.44 \pm 0.07) \times 10^3$ RFU per millimolar concentration. Subsequently the unknown

thiolated gelatin is assayed and a resulting intensity is found per weighed amount.

Due to the DS-dependent (unknown) REC-SH molecular weight of a sample a straightforward conversion of sample weight to sample moles is impossible. Instead, we have a set of two equations with two unknowns, the REC-SH molecular weight (Z) and the number of modified lysine per molecule REC (x) that can be solved for both.

Equations to solve:

[Chem.13]

$$Z = P + x\,Q \qquad \text{Eq 3.}$$

where Z is the modified REC molecular weight, P is the unmodified REC molecular weight, Q is the REC molecular weight increase due to the coupling of x functional groups per REC molecule. This depends on the thiolating reagent used (SH1 or SH2). The concentration of (OPA-labeled) amines in solution as a result of the residual primary amines per chain ($N_f$) is given by:
[Chem.14]

$$N_f = \left(N_{LysNH2,mol} - x\right) \frac{W}{Z\,V} \qquad \text{Eq 4.}$$

where W is the modified REC dry mass, W/V is the sample concentration in grams per liter at final dilution and $N_{LysNH2,mol}$ equals the number of lysine amines per mole REC (unmodified). This value is a designed parameter that depends on the amino acid sequence of the REC. For REC1 $N_{LySNH2,mol}$ equals 33, as can be seen in the listed sequences.

Equation 4, using the measured emission, rearranges to:
[Chem.15]

$$Em_{460} = \left(N_{LysNH2,mol} - x\right) RFU_{mol} \frac{W}{Z\,V} \qquad \text{Eq 5.}$$

where $RFU_{mol}$ is the molar emission intensity of OPA-coupled Lys groups. $Em_{460}$ equals the blank corrected sample emission (RFU). The unknowns are Z and x in the equations 3 and 5.

Then isolating x via some simple math results in the solution for x in equation 6.
[Chem. 16]

$$x = \frac{\left(N_{LysNH2,mol} RFU_{mol} \frac{W}{V} - Em_{460} P\right)}{Em_{460}\,Q + RFU_{mol} \frac{W}{V}} \qquad \text{Eq 6.}$$

With x we have the modification level of the MG (DS% = x/ $N_{LysNH2,mol}$). Then use the solution for x to find the modified REC molecular weight Z using equation 3.

TNBS assay protocol

A large number of TNBS assay protocols have been published since the first synthesis of TNBS by Okuyama and Satake in 1960. A preferred protocol is that published by Bubnis and Ofner in Analytical Biochemistry, 1992, Vol.207, page 129-133. In this protocol a known dry weight of the gelatin is mixed in solution with an excess of the TNBS reagent at an alkaline pH 8.5 for 4 hours at 40°C. During that time the TNBS reagent reacts with all lysine ε-amine groups and labels them with a chromophore that can be detected and quantified at a wavelength of 346nm using a UV/VIS spectrophotometer. This protocol can be used for analysis of any gelatin or polysaccharide containing lysine, except when thiols are present, as these thiols compete with the lysine amines for the TNBS labelling agent.

Measurement of MAWM by GPC-MALS

The theoretical molecular weights MW of recombinant gelatins can be easily obtained by a person skilled in the art by inspecting the amino acid sequence and summing all residues. The modified gelatin MW can then be derived by correcting the REC MW for the number of modified lysine residues with the thiolating reagent used. The actual recombinant gelatin production process however yields the recombinant gelatins with a slight number of cleaved

fragments resulting in some macromolecular polydispersity. This polydispersity can be detected and quantified by Gel Permeation Chromatography (GPC) equipped with a Multi-Angle Light Scattering detector (MALS). Such a detector measures the absolute molecular weight of each component in the chromatogram. It is then straightforward for a person skilled in the art to derive the number-averaged molecular weight NAMW and the weight-averaged molecular weight WAMW. Furthermore, it is common practice to describe the polydispersity using an index pD, defined by the relation pD = WAMW/NAMW.

Compressive Modulus

The compressive modulus E (kPa) of the hydrogels was determined after equilibration in PBS solution at 37°C by indenter testing with an Anton-Paar MCR-301 rheometer using an 8mm diameter flat indenter head. The gap size was lowered in small regular steps (strain) and the normal force (stress) was recorded. The straight section in the stress-strain curve was then used to find the compressive modulus.

Example 1

Preparation of Modified Gelatin MG1 using Thiolating Agent NAHCTL

A recombinant gelatin comprising more than 450μmoles/g of lysine per gram (REC1, 8.23g) was weighed into a 125ml Corning (Costar) polystyrene sterile bottle and CB10 (82.28g) was added. The bottle containing the mixture was shaken vigorously shaken before being placed in a 50°C water bath. Within about 15 to 20 minutes the recombinant gelatin had completely dissolved and the bottle was removed from the water bath. The solution of recombinant gelatin in PB10 was then transferred into the three-neck round-bottom flask. As a result of the transfer, 2.32g (2.6wt%) of the solution was lost. Therefore the amount of solution transferred into the 3-neck was 88.19g. Therefore the actual amount of recombinant gelatin transferred into the flask was 8.02g (not taking into account the initial moisture content). A solution of EDTA( 0.026g) in PBS (1.4cm$^3$) was added in order to complex-out any metals which might otherwise catalyze the oxidation of thiol groups. The flask was mounted in a water bath set at a temperature of 41°C and fitted with a magnetic stirrer (Komet 15), a thermometer and a Schlenk-type Liebig condenser topped by an adapter with glass stopcock.

The flask was purged with nitrogen gas continuously for 1 hour at 40°C. A thiolating agent (NAHCTL, 1.572g) was then added while stirring the flask contents at 5000rpm at 40°C and continuing the purging with nitrogen gas. After 3 hours reaction time, the flask was removed from the water bath and the pH of the flask contents were reduced in pH to 5.1 (while purging was continued) using hydrochloric acid (1M). The contents of the flask were then diluted with purified water (about 150cm$^3$). The flask contents were stirred briefly and vacuum filtered using a sterile 0.22mu PES bottle top filter into a 250ml Corning bottle to give a filtrate.

Dialysis

The filtrate was transferred into a dialysis tube (30cm long and with a 14 kDa molecular weight cut off, obtained from Spectra/Por RC) and the dialysis tubing cellulose membrane (D9402, avg. flat width 76mm (3.0 in.), Sigma) was put in a 10L glass beaker containing 10 litres of nitrogen-purged deionized water at 35°C. The nitrogen purging was continued with some heating of the water present in the beaker to maintain a water temperature of approximately 35°C. For a total of 2 days, the water present in the beaker was replaced every 3 hours with 10 litres of nitrogen-purged water.

Freeze-drying

The contents of the dialysis tube were collected in freezing containers, frozen at - 40°C and lyophilized in a Zirbus Sublimator for 4 days at -15°C. The resultant, dry sample of modified gelatin MG1 was then collected, weighed, split into several sterile twirl bags and stored in a freezer at -20°C. The modified gelatin MG1 was obtained as a white fibrous cake-like material in a yield of 90%.

MG1 was found to contain 584.7μmoles/g of thiol groups derived from the thiolating agent.

Example 2

Preparation of Modified Gelatin MG2 using the Thiolating Agent 2-IT

REC1 (8.06g) was loaded into a 125ml Corning bottle. Buffer CB10 (76.45g) was then added and the bottle was then placed in a 50°C bath. The bottle was regularly shaken, manually, until a homogeneous solution of REC1 in the buffer was obtained. EDTA (0.022g) was added and the mixture was then transferred into a 100ml three-neck round-bottom flask

provided with a nitrogen purging inlet. A magnetic stirrer (type comet) was placed in the flask along with a thermometer. A Schlenk-type Liebig condenser was fitted to the flask, topped by an adapter with glass stopcock. After purging the flask with nitrogen gas for at least an hour, with stirring, a solution of thiolating agent (2-IT,897.6mg) in buffer PB8 (7.0g) was added to the flask with stirring at a temperature of 41°C. After 1 hour stirring at 41°C, with continuous purging with damp nitrogen gas, the contents of the flask (90.52g) were collected and diluted with $N_2$-purged sterile water (209.75g) at room temperature then vacuum-filtered using a 0.22mu Corning bottle top filter to remove any solids. The filtrate had a volume of approximately 300$cm^3$.

Dialysis

The filtrate was transferred into a dialysis tube (30cm long and with a 14 kDa molecular weight cut off, obtained from Spectra/Por RC) and the dialysis tubing cellulose membrane (D9402, avg. flat width 76mm (3.0 in.), Sigma) was put in a 10L glass beaker containing 10 litres of nitrogen-purged deionized water at 35°C. The nitrogen purging was continued with some heating of the water present in the beaker to maintain a water temperature of approximately 35°C. For a total of 2 days, the water present in the beaker was replaced every 3 hours with 10 litres of nitrogen-purged water.

Freeze-drying

The contents of the dialysis tube were collected in freezing containers, frozen at - 40°C and lyophilized in a Zirbus Sublimator for 4 days at -15°C. The resultant, dry sample of Modified gelatin MG2 was then collected, weighed, split into several sterile twirl bags and stored in a freezer at -20°C. Modified gelatin MG2 was obtained as a white fibrous cake-like material in a yield of 93%.

MG2 was found to contain 605.2μmoles/g of thol groups derived from the thiolating agent.

Examples 3 to 10 and Comparative Examples CEx1 to CEx4

The general methods of Examples 1 were repeated except that recombinant gelatin and thiolating agents indicated in Table 1 below were used in the amounts indicated to give the modified gelatins MG3 to MG10 and comparative modified gelatins CMG1 to CMG4. In Table 1, the recombinant gelatins thiolated using NAHCL followed the general method of Example 1 above and the recombinant gelatins thiolated using 2-IT followed the general method of Example 2 above. For completeness Table 1 also includes MG1 and MG2.

[Table 1]

| Table 1 - Reaction of Recombinant gelatins with Thiolating Agents | | | | |
|---|---|---|---|---|
| Example | Recombinant gelatin and amount of lysine present (μmoles/g) | Thiolating agent and amount used per g of solid, dry recombinant gelatin | Amount of thiol groups in resultant modified gelatin (μmoles/g) * | Name of resultant modified gelatin |
| 1 | REC1 (644.7) | NAHCTL (204.0mg) | 584.7 | MG1 |
| 2 | REC1 (644.7) | 2-IT (120.8mg) | 605.2 | MG2 |
| 3 | REC2-(646.2) | NAHCTL (204.5mg) | 585.9 | MG3 |
| 4 | REC2 (646.2) | 2-IT (121.1mg) | 606.6 | MG4 |
| 5 | REC1 (644.7) | NAHCTL (102.0mg) | 306.6 | MG5 |
| 6 | REC1 (644.7) | NAHCTL (40.8mg) | 126.3 | MG6 |
| 7 | REC1 (644.7) | NAHCTL (12.2mg) | 38.4 | MG7 |
| 8 | REC1 (644.7) | 2-IT (60.4mg) | 312.2 | MG8 |
| 9 | REC1 (644.7) | 2-IT (24.2mg) | 127.3 | MG9 |
| 10 | REC1 (644.7) | 2-IT (7.2mg) | 38.5 | MG10 |
| CEx1 | CREC1 (400.3) | NAHCTL (126.7mg) | 376.4 | CMG1 |
| CEx2 | CREC1 (400.3) | 2-IT (75.0mg) | 384.8 | CMG2 |

(continued)

| Table 1 - Reaction of Recombinant gelatins with Thiolating Agents | | | | |
|---|---|---|---|---|
| Example | Recombinant gelatin and amount of lysine present (μmoles/g) | Thiolating agent and amount used per g of solid, dry recombinant gelatin | Amount of thiol groups in resultant modified gelatin (μmoles/g) * | Name of resultant modified gelatin |
| CEx3 | CREC2 (441.6) | NAHCTL (139.7mg) | 412.6 | CMG3 |
| * This is also the amount of lysine residues comprising a pendent side chain carrying a thiol group. | | | | |

Example 11 - Preparation of a Hydrogel (HG1)

Preparation of hydrogels

Stage 1 - Preparation of a solution containing the modified gelatin MG1

A sample of MG1 (115.6mg) was pushed down using tweezers into a Greiner centrifuge conical tube of capacity 15cm$^3$. PBS buffer (0.751cm$^3$) was added to the tube in order to fully wet the sample of MG1. The tube was then centrifuged at 18,000G for 2 minutes in order to fully dissolve MG1 in the PBS buffer and force out any entrapped gas bubbles. The mixture was then vortexed by an IKA Vortex orbital shaker for 5 seconds to provide a homogenous, clear solution. Sodium hydroxide solution (1.0M, 18.2μL) was added to obtain a mixture having a pH in the range 7.2 to 7.6. The mixture was again vortexed once more in order to provide a solution of MG1 for crosslinking ("Solution 1").

Stage 2 - Preparation of a solution containing a Crosslinking Agent

A sample of PEG-AC4 (84.5mg) was dissolved in PBS buffer (1.022cm$^3$) which had been purged with nitrogen gas to remove oxygen gas. The resultant solution of crosslinking agent is herein referred to as "Solution 2".

Stage 3 - Preparation of Hydrogel HG1

Solution 2 (1.111g) was added to Solution 1 (889mg) under an atmosphere of nitrogen gas and the two solutions were mixed well using an IKA Vortexer. The resultant mixture was then placed into a mould, covered to prevent drying out and stored at 37°C for 16 hours. Subsequently the hydrogel was immersed in PBS solution at 37°C for 24 more hours to allow equilibration. The resultant hydrogel, HG1, had the properties indicated in Table 2 below.

Examples 12 to 26 and Comparative Examples CEx5 to CEx11 - Preparation of further Hydrogels

The general method of Example 11 was repeated except that the modified gelatins and crosslinking agents indicated in Table 2 below were used in the amounts indicated to give the hydrogels HG2 to HG13 and comparative modified hydrogels CHG1 and CHG2 as indicated in the final column. For completeness Table 2 also includes HG1. Each hydrogel comprised about 90wt% water and 10wt% of a compound formed by reaction of the relevant modified gelatin and crosslinking agent.

[Table 2]

| Table 2 - Reaction of Modified Gelatins with Crosslinking Agents to form Compounds and Hydrogels | | | | | |
|---|---|---|---|---|---|
| Example | Modified gelatin and amount (mg) | Crosslinking agent and amount (mg) | Time t taken for the mod. gelatin to react with the crosslinking agent (gel-time§) (minutes) | Release rate R‡ of encapsulated FITC-dextran (150 kD) | Name of resultant Hydrogel |
| 11 | MG1 (131.1mg) | AC-PEG-MAL 1 ( 68.6mg) | A | B | HG1 |
| 12 | MG1 (165.4mg) | AC-PEG-MAL 1 ( 34.6mg) | A | B | HG2 |

(continued)

| Table 2 - Reaction of Modified Gelatins with Crosslinking Agents to form Compounds and Hydrogels | | | | | |
|---|---|---|---|---|---|
| Example | Modified gelatin and amount (mg) | Crosslinking agent and amount (mg) | Time t taken for the mod. gelatin to react with the crosslinking agent (gel-time§) (minutes) | Release rate R‡ of encapsulated FITC-dextran (150 kD) | Name of resultant Hydrogel |
| 13 | MG5 (144.6mg) | PEG-AC4 (55.4mg) | A | B | HG3 |
| 14 | MG5 (132.1mg) | AC-PEG-AC 1 ( 67.9mg) | B | B | HG4 |
| 15 | MG5 (131.5mg) | AC-PEG-MAL 1 (68.5mg) | A | B | HG5 |
| 16 | MG5 (173.4mg) | PEG-AC4 (26.6mg) | A | B | HG6 |
| 17 | MG5 (165.5mg) | AC-PEG-MAL 1 (34.5mg) | A | B | HG7 |
| 18 | MG6 (172.7mg) | PEG-AC4 (27.3mg) | A | B | HG8 |
| 19 | MG6 (164.6mg) | AC-PEG-MAL 1 (35.4mg) | A | B | HG9 |
| 20 | MG6 (188.1mg) | PEG-AC4 (11.9mg) | A | B | HG10 |
| 21 | MG6 (184.2mg) | AC-PEG-MAL 1 (15.8mg) | A | A | HG11 |
| 22 | MG6 (177.6mg) | AC-PEG-MAL 1 (22.4mg) | A | A | HG12 |
| 23 | MG2 (180.7mg) | MAL-PEG-MAL (19.3mg) | B | ND | HG14 |
| 24 | MG8 (189.6mg) | MAL-PEG-MAL (10.4mg) | B | ND | HG15 |
| 25 | MG9 (195.6mg) | MAL-PEG-MAL (4.4mg) | A | ND | HG16 |
| 26 | MG4 (180.7mg) | MAL-PEG-MAL (19.3mg) | B | ND | HG17 |
| CEx5 | MG7 (187.7mg) | AC-PEG-MAL 1 (12.3mg) | C | !! | CHG1 |
| CEx6 | CMG1 (161.0mg) | PEG-AC4 (39.0mg) | C | C | CHG2 |
| CEx7 | CMG1 (176.7mg) | AC-PEG-MAL 1 (23.3mg) | C | !! | CHG3 |
| CEx8 | MG10 (198.7mg) | MAL-PEG-MAL (1.35mg) | B | ND | CHG4 |

(continued)

| Table 2 - Reaction of Modified Gelatins with Crosslinking Agents to form Compounds and Hydrogels | | | | | |
|---|---|---|---|---|---|
| Example | Modified gelatin and amount (mg) | Crosslinking agent and amount (mg) | Time t taken for the mod. gelatin to react with the crosslinking agent (gel-time§) (minutes) | Release rate R‡ of encapsulated FITC-dextran (150 kD) | Name of resultant Hydrogel |
| CEx9 | CMG1 (187.6mg) | MAL-PEG-MAL (12.4mg) | C | ND | CHG5 |
| CEx10 | CMG5 (193.4mg) | MAL-PEG-MAL (6.61mg) | C | ND | CHG6 |
| CEx11 | CMG4 (186.1mg) | MAL-PEG-MAL (13.9mg) | C | ND | CHG7 |

§: Range of gel-times t:
A: 10min < t < 45min (most preferred);
B: 45min < t < 2h (preferred);
C: t < 10min or t > 2h (unacceptable).
‡: Encapsulated FITC-dextran 150kD release fraction from a 1mm thick hydrogel at 37°C;
A: R > 90% (most preferred);
B: 75% < R < 90% (preferred);
C: R < 75% (unacceptable);
!!: No gelation.
ND means not determined.

Testing of the Hydrogels

Transparency

The hydrogels of the present invention were found to have excellent transparency.

Compressive Modulus

The Compressive Modulus of the hydrogels listed in Table 3 below was determined by the method described above and the results are shown in Table 3 below:

[Table 3]

| Table 3 - Hydrogel Compressive Modulus | | | | |
|---|---|---|---|---|
| Hydrogel | Original gelatin and μmoles/g of lysine present therein | Amount of thiol groups in the modified gelatin used to make the hydrogel (μmoles/g) * | The standard deviation of lys-lys distances in the gelatin, $SD_{Lys}$ *(AA)* | Compress. modulus E (kPa) of the hydrogel |
| HG14 | REC1 (644.7) | 605 | 11.7 | A |
| HG15 | REC1 (644.7) | 312 | 11.7 | B |
| HG16 | REC1 (644.7) | 127 | 11.7 | B |
| HG17 | REC2 (646.2) | 607 | 11.8 | A |
| CHG4 | REC1 (644.7) | 38 | 11.7 | C |
| CHG5 | CREC1 (400.3) | 376 | 17.4 | C |

(continued)

| Table 3 - Hydrogel Compressive Modulus | | | | |
|---|---|---|---|---|
| Hydrogel | Original gelatin and μmoles/g of lysine present therein | Amount of thiol groups in the modified gelatin used to make the hydrogel (μmoles/g) * | The standard deviation of lys-lys distances in the gelatin, $SD_{Lys}$ *(AA)* | Compress. modulus E (kPa) of the hydrogel |
| CHG6 | CREC1 (400.3) | 194 | 17.4 | C |
| Note 1: The standard deviation of lys-lys distances in the gelatin, $SD_{Lys}$ (AA) was calculated as described above in the description.<br>Note 2: In Table 3,<br>A: 200 < E < 600 kPa (most preferred);<br>B: 110 < E < 200 kPa; and<br>C: E < 110 kPa.<br>Note 3: This is also the amount of lysine residues comprising a pendent side chain carrying a thiol group. | | | | |

Release Properties of the Inventive and Comparative Hydrogels

The hydrogels described in Table 2 above were prepared in the presence of a water-soluble, fluorescent marker (FITC-dextran 150kD, 4kD). The hydrogels comprising the marker were then placed in PBS solution at 37°C and the rate at which the marker was released from the hydrogel over time was measured by fluorescence spectroscopy. The experiments showed that the hydrogels according to the invention had faster release properties than the comparative hydrogels derived from recombinant gelatins containing less than the claimed amount of lysine. This means the hydrogels of the present invention were better in enabling diffusion of solutes through the hydrogel network than the comparative Examples. Thus the hydrogels of the present invention are more suitable for medical uses such as cell encapsulation.

**Claims**

**1.** A modified gelatin comprising:

(a) lysine residues; and
(b) lysine residues comprising a pendent side chain carrying a thiol group;

wherein the modified gelatin comprises at least 50μmoles/g of component (b) and at least 450μmoles/g in total components (a) and (b).

**2.** A modified gelatin according to claim 1 which comprises components (a) and (b) at an average distance of 17 amino acids with a distance standard deviation of 12 amino acids, and/or
wherein the standard deviation of the distances between components of type (a), between components of type (b) and between components of type (a) and (b) is less than 14 amino acid residues.

**3.** A modified gelatin according to claim 1 or 2 which has a weight-averaged molecular weight from 15,000 to 80,000 Daltons, and/or
which has an isoelectric point of at least 5.

**4.** A modified gelatin according to any one of claims 1 to 3 in which the percentage of RGD motifs relative the total number of amino acids present in the recombinant gelatin is at least 0.4%, and/or

wherein the modified gelatin comprises at least two RGD sequences per 250 amino acids, and/or
wherein the modified gelatin is free from cysteine.

**5.** A process for preparing a modified gelatin comprising reacting a recombinant gelatin comprising at least 450μmoles/g of lysine with a thiolating agent, wherein the modified gelatin comprises at least 50μmoles/g of thiol groups derived from the thiolating agent.

6. A process according to claim 5 wherein the thiolating agent comprises a 2-iminothiolane and/or DL- N-acetylhomocysteine thiolactone and/or a salt thereof.

7. A modified gelatin obtainable by a process according to claim 5 or 6.

8. A hydrogel obtainable by reacting a modified gelatin according to any one of claims 1 to 4 or claim 7 with a crosslinking agent using a click-reaction.

9. A hydrogel obtainable by reacting a modified gelatin according to claim 8 where the click-reaction is a thiol-Michael addition reaction.

10. A hydrogel according to claim 8 or 9 wherein the crosslinking agent comprises at least two ethylenically unsaturated groups, and/or

wherein the crosslinking agent comprises at least one maleimide group, and/or
wherein the crosslinking agent comprises at least one maleimide group and at least one acrylate group, and/or
wherein the crosslinking agent comprises one and only one maleimide group and one and only one acrylate group, and/or
wherein the crosslinking agent has an Mw of from 2,000 to 30,000 Daltons.

11. A hydrogel according to any one of claims 8 to 10 wherein the number of moles of crosslinking agent which are added to the modified gelatin are insufficient to react with all of the thiol groups present in the modified gelatin, and/or wherein the number of moles of crosslinking agent which are added to the modified gelatin are sufficient to react with at most 95% of the thiol groups present in the modified gelatin.

12. A hydrogel according to any one of claims 8 to 11 which comprises from 0.5 to 15μmoles/g of thiol groups, and/or

which comprises 65 to 97.5 wt% of water, and/or
which has a solids content of up to 10wt%.

13. A process for preparing hydrogels, comprising reacting a modified gelatin according to any one of claims 1 to 4 or claim 7 with a crosslinking agent using a click-reaction.

14. A kit of parts comprising:

a. a modified gelatin according to any one of claims 1 to 4 or claim 7; and
b. a crosslinking agent.

15. A kit of parts according to claim 14 wherein the crosslinking agent comprises one and only one maleimide group and one and only one acrylate group, and/or wherein the kit further comprises instructions to crosslink the modified gelatin with the crosslinking agent.

## Patentansprüche

1. Modifizierte Gelatine, umfassend:

(a) Lysinreste und
(b) Lysinreste, die eine anhängige Seitenkette umfassen, die eine Thiolgruppe aufweisen,

worin die modifizierte Gelatine mindestens 50 μmol/g der Komponente (b) und insgesamt mindestens 450 μmol/g der Komponenten (a) und (b) umfasst.

2. Modifizierte Gelatine gemäß Anspruch 1, die die Komponenten (a) und (b) in einem mittleren Abstand von 17 Aminosäuren mit einem Standardabweichungs-Abstand von 12 Aminosäuren umfasst, und/oder
worin die Standardabweichung der Abstände zwischen den Komponenten vom Typ (a), zwischen den Komponenten vom Typ (b) und zwischen den Komponenten von Typ (a) und (b) weniger als 14 Aminosäurereste beträgt.

3. Modifizierte Gelatine gemäß Anspruch 1 oder 2, die ein gewichtsgemitteltes Molekulargewicht von 15.000 bis 80.000 Dalton aufweist, und/oder
die einen isoelektrischen Punkt von mindestens 5 aufweist.

4. Modifizierte Gelatine gemäß irgendeinem der Ansprüche 1 bis 3, worin der Prozentanteil des RGD-Motivs, relativ zur Gesamtzahl von Aminosäuren, die in der rekombinanten Gelatine vorliegen, mindestens 0,4 % beträgt, und/oder worin die modifizierte Gelatine mindestens zwei RGD-Sequenzen je 250 Aminosäuren umfasst, und/oder worin die modifizierte Gelatine kein Cystein aufweist.

5. Verfahren zur Herstellung einer modifizierten Gelatine, umfassend das Umsetzen einer rekombinanten Gelatine, umfassend mindestens 450 μmol/g von Lysin, mit einem Thiolierungsmittel, worin die modifizierte Gelatine mindestens 50 μmol/g Thiolgruppen umfasst, die aus dem Thiolierungsmittel stammen.

6. Verfahren gemäß Anspruch 5, worin das Thiolierungsmittel ein 2-Iminothiolan und/oder DL-N-Acetylhomocysteinthiolakton und/oder ein Salz hiervon umfasst.

7. Modifizierte Gelatine, erhältlich durch ein Verfahren gemäß Anspruch 5 oder 6.

8. Hydrogel, erhältlich durch Umsetzen einer modifizierten Gelatine gemäß irgendeinem der Ansprüche 1 bis 4 oder gemäß Anspruch 7 mit einem Vernetzungsmittel unter Verwendung einer Click-Reaktion.

9. Hydrogel, erhältlich durch Umsetzen einer modifizierten Gelatine gemäß Anspruch 8, worin die Click-Reaktion eine Thiol-Michael-Additionsreaktion ist.

10. Hydrogel gemäß Anspruch 8 oder 9, worin das Vernetzungsmittel zumindest zwei ethylenisch ungesättigte Gruppen umfasst, und/oder

    worin das Vernetzungsmittel zumindest eine Maleimidgruppe umfasst, und/oder
    worin das Vernetzungsmittel zumindest eine Maleimidgruppe und zumindest eine Acrylatgruppe umfasst, und/oder
    worin das Vernetzungsmittel eine, und nur eine, Maleimidgruppe und eine, und nur eine, Acrylatgruppe umfasst, und/oder
    worin das Vernetzungsmittel ein Mw von 2.000 bis 30.000 Dalton aufweist.

11. Hydrogel gemäß irgendeinem der Ansprüche 8 bis 10, worin die Molzahl des Vernetzungsmittels, die zu der modifizierten Gelatine zugegeben werden, unzureichend ist, um mit allen Thiolgruppen, die in der modifizierten Gelatine vorliegen, zu reagieren, und/oder
worin die Molzahl des Vernetzungsmittels, die zu der modifizierten Gelatine zugegeben wird, ausreichend ist, um mit mindestens 95 % der Thiolgruppen, die in der modifizierten Gelatine vorliegen, zu reagieren.

12. Hydrogel gemäß irgendeinem der Ansprüche 8 bis 11, welches von 0,5 bis 15 μmol/g an Thiolgruppen umfasst und/oder

    welches 65 bis 97,5 Gew.-% Wasser umfasst und/oder
    welches einen Feststoffgehalt von bis zu 10 Gew.-% aufweist.

13. Verfahren zur Herstellung von Hydrogelen, umfassend das Umsetzen einer modifizierten Gelatine gemäß irgendeinem der Ansprüche 1 bis 4 oder gemäß Anspruch 7 mit einem Vernetzungsmittel unter Verwendung einer Click-Reaktion.

14. Set von Teilen, umfassend:

    a. eine modifizierte Gelatine gemäß irgendeinem der Ansprüche 1 bis 4 oder gemäß Anspruch 7; und
    b. ein Vernetzungsmittel.

15. Set von Teilen gemäß Anspruch 14, worin das Vernetzungsmittel eine, und nur eine, Maleimidgruppe umfasst und eine, und nur eine, Acrylatgruppe umfasst, und/oder
worin das Set ferner Instruktionen umfasst, um die modifizierte Gelatine mit dem Vernetzungsmittel zu vernetzen.

## Revendications

1. Gélatine modifiée comprenant :

   (a) des résidus de lysine ; et
   (b) des résidus de lysine comprenant une chaîne latérale pendante portant un groupe thiol ;

   dans laquelle la gélatine modifiée comprend au moins 50 μmoles/g de composant (b) et au moins 450 μmoles/g au total de composants (a) et (b).

2. Gélatine modifiée selon la revendication 1 qui comprend les composants (a) et (b) à une distance moyenne de 17 acides aminés avec un écart type de distance de 12 acides aminés, et/ou
   dans laquelle l'écart type des distances entre les composants du type (a), entre les composants du type (b) et entre les composants du type (a) et (b) est inférieur à 14 résidus d'acides aminés.

3. Gélatine modifiée selon la revendication 1 ou la revendication 2 qui présente un poids moléculaire moyen en poids de 15 000 à 80 000 Daltons, et/ou
   qui a un point isoélectrique d'au moins 5.

4. Gélatine modifiée selon l'une quelconque des revendications 1 à 3 dans laquelle le pourcentage d'unités RGD par rapport au nombre total d'acides aminés présents dans la gélatine recombinante est d'au moins 0,4 %, et/ou

   dans laquelle la gélatine modifiée comprend au moins deux séquences RGD pour 250 acides aminés, et/ou
   dans laquelle la gélatine modifiée est exempte de cystéine.

5. Procédé de préparation d'une gélatine modifiée comprenant la mise en réaction d'une gélatine recombinante comprenant au moins 450 μmoles/g de lysine avec un agent de thiolation, dans lequel la gélatine modifiée comprend au moins 50 μmoles/g de groupes thiol dérivés de l'agent de thiolation.

6. Procédé selon la revendication 5 dans lequel l'agent de thiolation comprend un 2-iminothiolane et/ou une DL-N-acétylhomocystéine thiolactone et/ou un sel de ceux-ci.

7. Gélatine modifiée pouvant être obtenue par un procédé selon la revendication 5 ou la revendication 6.

8. Hydrogel pouvant être obtenu par mise en réaction d'une gélatine modifiée selon l'une quelconque des revendications 1 à 4 ou la revendication 7 avec un agent de réticulation en utilisant une réaction clic.

9. Hydrogel pouvant être obtenu par mise en réaction d'une gélatine modifiée selon la revendication 8 où la réaction clic est une réaction d'addition thiol-Michael.

10. Hydrogel selon la revendication 8 ou la revendication 9 dans lequel l'agent de réticulation comprend au moins deux groupes éthyléniquement insaturé, et/ou

    dans lequel l'agent de réticulation comprend au moins un groupe maléimide, et/ou
    dans lequel l'agent de réticulation comprend au moins un groupe maléimide et au moins un groupe acrylate, et/ou
    dans lequel l'agent de réticulation comprend un et un seul groupe maléimide et un et un seul groupe acrylate, et/ou
    dans lequel l'agent de réticulation a un Mw de 2 000 à 30 000 Daltons.

11. Hydrogel selon l'une quelconque des revendications 8 à 10 dans lequel le nombre de moles d'agent de réticulation qui sont ajoutées à la gélatine modifiée est insuffisant pour réagir avec l'ensemble de groupes thiol présents dans la gélatine modifiée, et/ou
    dans lequel le nombre de moles d'agent de réticulation qui sont ajoutées à la gélatine modifiée est suffisant pour réagir avec au plus 95 % des groupes thiol présents dans la gélatine modifiée.

12. Hydrogel selon l'une quelconque des revendications 8 à 11 qui comprend de 0,5 à 15 μmoles/g de groupes thiol, et/ou

    qui comprend 65 à 97,5 % en poids d'eau, et/ou

qui présente une teneur en matières solides de jusqu'à 10 % en poids.

**13.** Procédé de préparation d'hydrogels, comprenant la mise en réaction d'une gélatine modifiée selon l'une quelconque des revendications 1 à 4 ou la revendication 7 avec un agent de réticulation en utilisant une réaction clic.

**14.** Kit de pièces comprenant :

a. une gélatine modifiée selon l'une quelconque des revendications 1 à 4 ou la revendication 7 ; et
b. un agent de réticulation.

**15.** Kit de pièces selon la revendication 14 dans lequel l'agent de réticulation comprend un et un seul groupe maléimide et un et un seul groupe acrylate, et/ou dans lequel le kit comprend en outre des instructions pour réticuler la gélatine modifiée avec l'agent de réticulation.

[Fig. 1]

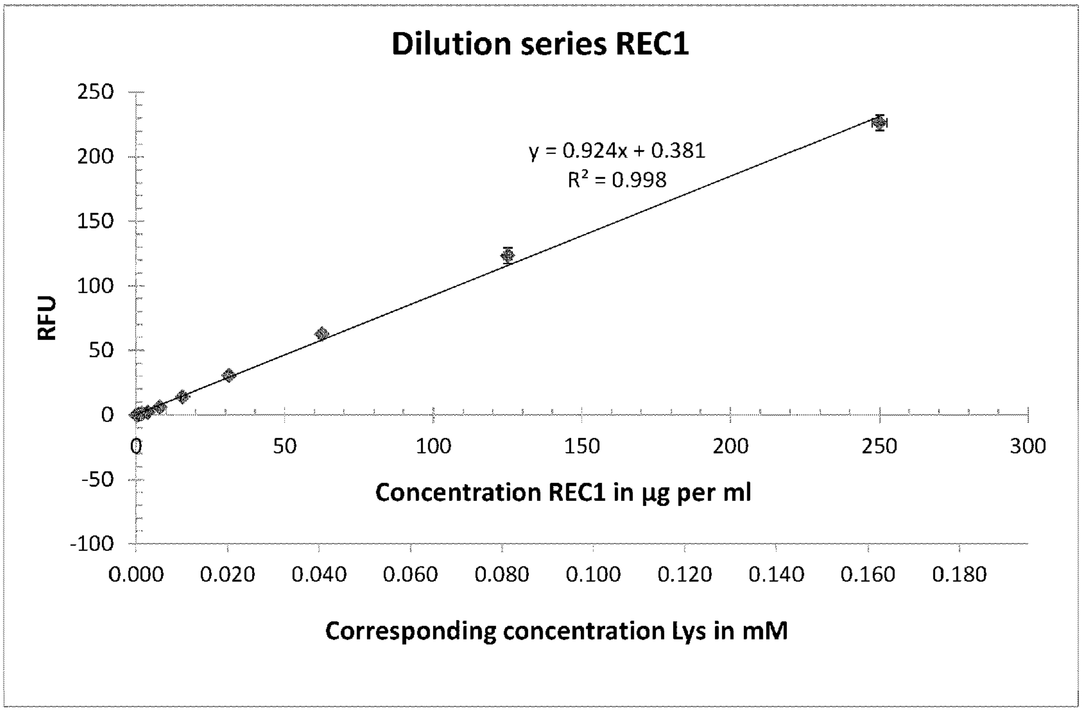
Dilution series REC1
y = 0.924x + 0.381
$R^2$ = 0.998
RFU
250
200
150
100
50
0
-50
-100
0
50
100
150
200
250
300
Concentration REC1 in µg per ml
0.000
0.020
0.040
0.060
0.080
0.100
0.120
0.140
0.160
0.180
Corresponding concentration Lys in mM

[Fig. 2]

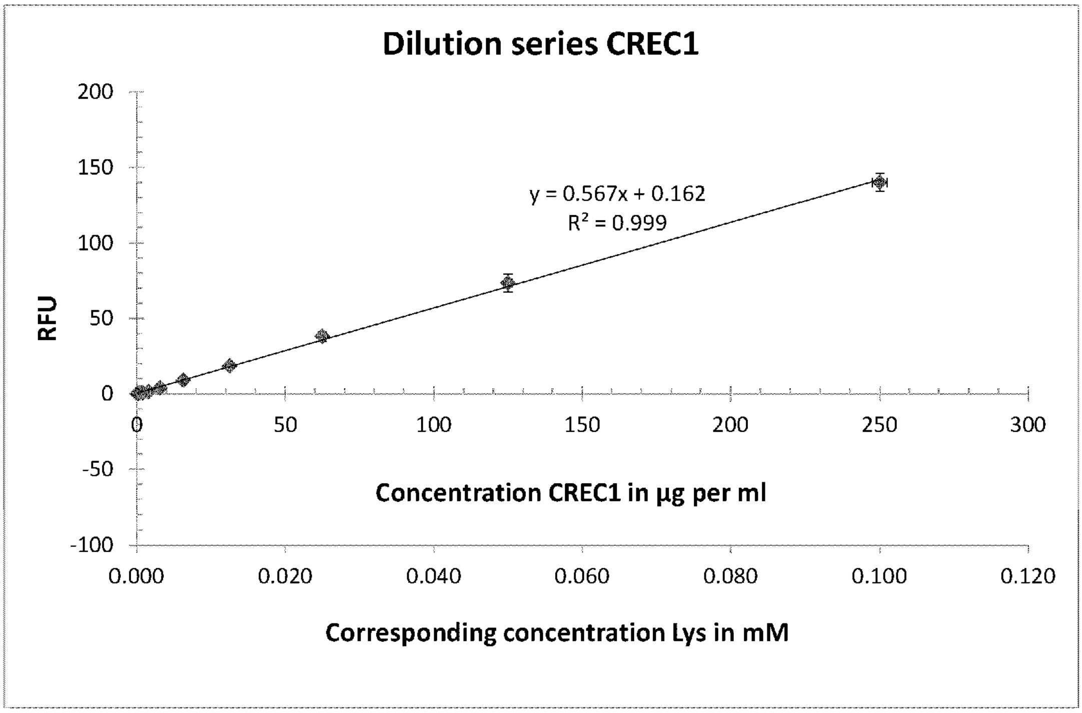
Dilution series CREC1
y = 0.567x + 0.162
$R^2$ = 0.999
RFU
200
150
100
50
0
-50
-100
0
50
100
150
200
250
300
Concentration CREC1 in µg per ml
0.000
0.020
0.040
0.060
0.080
0.100
0.120
Corresponding concentration Lys in mM

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008103041 A
- EP 0926543 A
- EP 1014176 A
- WO 0134646 A
- WO 200803041 A
- EP 2902413 A
- US 8101205 B


### Non-patent literature cited in the description


- **VAN VLIERBERGHE et al.** *European Polymer Journal*, 2011, vol. 47, 1039-1047
- **M.W.T. WERTEN et al.** High yield secretion of recombinant gelatins by Pichia pastoris. *Yeast*, 1999, vol. 15, 1087-1096
- CRC Handbook of Chemistry and Physics. CRC Press LLC, 1997
- **BUBNIS** ; **OFNER**. *Analytical Biochemistry*, 1992, vol. 207, 129-133